(19) **Europäisches Patentamt / European Patent Office / Office européen des brevets**

(11) **EP 2 734 217 B2**

(12) ## NEUE EUROPÄISCHE PATENTSCHRIFT
Nach dem Einspruchsverfahren

(45) Veröffentlichungstag und Bekanntmachung des Hinweises auf die Entscheidung über den Einspruch:
**10.06.2026 Patentblatt 2026/24**

(45) Hinweis auf die Patenterteilung:
**02.12.2020 Patentblatt 2020/49**

(21) Anmeldenummer: **12735583.2**

(22) Anmeldetag: **19.07.2012**

(51) Internationale Patentklassifikation (IPC):
**A61K 38/01** *(2006.01)* **A61L 27/00** *(2006.01)*

(52) Gemeinsame Patentklassifikation (CPC):
**A23L 33/40; A23L 33/175; A61K 38/01; A61P 3/02;**
A61K 38/00

(86) Internationale Anmeldenummer:
**PCT/EP2012/064220**

(87) Internationale Veröffentlichungsnummer:
**WO 2013/014064 (31.01.2013 Gazette 2013/05)**

(54) **PRODUKT ZUR PARENTERALEN ERNÄHRUNG VON ADIPÖSEN INTENSIVPATIENTEN**

PRODUCT FOR PARENTERAL FEEDING OF OBESE INTENSIVE CARE PATIENTS

PRODUIT DESTINÉ À L'ALIMENTATION PARENTÉRALE DE PATIENTS À FORT PANNICULE ADIPEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.07.2011 EP 11175036**
**22.07.2011 US 201161510512 P**

(43) Veröffentlichungstag der Anmeldung:
**28.05.2014 Patentblatt 2014/22**

(73) Patentinhaber: **Fresenius Kabi Deutschland GmbH**
**61352 Bad Homburg (DE)**

(72) Erfinder:
• **ABELE, Rosa**
**61462 Königstein (DE)**
• **SCHLOTZER, Ewald**
**61440 Oberursel (DE)**

(74) Vertreter: **Fresenius Kabi Deutschland GmbH**
**Patent Department**
**Borkenberg 14**
**61440 Oberursel (DE)**

(56) Entgegenhaltungen:
WO-A1-2004/056196    WO-A1-2005/060952
WO-A1-2005/096845    WO-A2-2007/080149
WO-A2-96/39051    WO-A2-96/39053

EP 2 734 217 B2

## Beschreibung

Intensivpatienten sind in der Regel nicht in der Lage, selbständig Nahrung aufzunehmen und müssen daher künstlich ernährt werden. Bei vielen Intensivpatienten besteht eine Indikation zur parenteralen Ernährung, da eine orale oder enterale Ernährung nicht oder nicht in ausreichendem Ausmaß erfolgen kann oder kontraindiziert ist. Bei der parenteralen Ernährung wird die Nahrung über die Blutbahn unter Umgehung des Magen-Darm-Trakts verabreicht.

Besondere Ansprüche an die parenterale Ernährung stellt die Gruppe der adipösen Intensivpatienten. Adipositas liegt bei starkem Übergewicht vor, das durch eine über das normale Maß hinausgehende Vermehrung des Körperfettes mit krankhaften Auswirkungen gekennzeichnet ist. Während sich adipöse Intensivpatienten bezüglich der Indikation zur parenteralen Ernährung im Allgemeinen nicht von normal- oder untergewichtigen Intensivpatienten unterscheiden, kommt es bei parenteral ernährten adipösen Intensivpatienten häufiger zum Auftreten von Komplikationen. Daneben ist der Schweregrad der bei parenteral ernährten Intensivpatienten auftretenden Komplikationen, beispielsweise im Vergleich zu normalgewichtigen parenteral ernährten Intensivpatienten, oft höher.

Dies ist zum Teil darauf zurückzuführen, dass eine adäquate parenterale Ernährung adipöser Intensivpatienten mit herkömmlichen parenteralen Ernährungslösungen des Stands der Technik nur schwer zu erreichen ist, da diese im Wesentlichen auf die Bedürfnisse von normal- oder untergewichtigen Patienten zugeschnitten sind.

Die Bedürfnisse adipöser Intensivpatienten hinsichtlich der Nahrungszusammensetzung unterscheiden sich aber stark von denen unter- oder normalgewichtiger Intensivpatienten. So benötigen adipöse Intensivpatienten andere Verhältnisse und Mengen der Makronährstoffe Aminosäuren, Kohlenhydrate und Lipide, unter Anderem da die Lipidverwertung, aber auch die Glukoseverwertung, bei dieser Patientengruppe oft gestört ist.

Zudem kann auch der Bedarf an Mikronährstoffen, wie Vitaminen und Spurenelementen, von dem unter- oder normalgewichtiger Intensivpatienten abweichen und ist oft erhöht.

Werden die von den Bedürfnissen unter- oder normalgewichtiger Intensivpatienten abweichenden Bedürfnisse adipöser Intensivpatienten nicht oder nicht in ausreichender Weise berücksichtigt, kann dies zu einem vermehrten Auftreten metabolischer oder infektiöser Komplikationen und somit zu einer erhöhten Morbidität und Mortalität der Patienten führen.

Im Stand der Technik werden parenterale Ernährungslösungen angeboten, die Aminosäuren, Lipide und Kohlenhydrate in bestimmten Mengen und Verhältnissen umfassen. Elektrolyte können in den Ernährungslösungen bereits enthalten sein oder dieser vor der Verabreichung zugesetzt werden. Spurenelemente und Vitamine werden den parenteralen Ernährungslösungen vor der Verabreichung zugesetzt oder getrennt von der parenteralen Ernährungslösung verabreicht. Diese parenteralen Ernährungslösungen des Stands der Technik sind nicht auf die Bedürfnisse adipöser Intensivpatienten ausgerichtet, sondern auf den Nährstoff- und Energiebedarf normal-oder untergewichtiger Intensivpatienten.

Auch die im Stand der Technik bereitgestellten Dosierungsempfehlungen parenteraler Ernährungslösungen sind nicht zur Ernährung adipöser Intensivpatienten ausgelegt. Beispielsweise nehmen Dosierungsanweisungen für parenterale Ernährungslösungen häufig Bezug auf das Körpergewicht des Intensivpatienten.

Das Körpergewicht als Bezugsgröße ist bei adipösen Patienten zur Ermittlung der benötigten Nahrungsmenge aber nicht gut geeignet, da es bei adipösen Intensivpatienten krankhaft erhöht ist. Eine Verabreichung von Nahrung basierend auf dem tatsächlichen Körpergewicht kann daher zu einer massiven Überversorgung mit Makronährstoffen führen. Zudem ist das Körpergewicht bei adipösen Intensivpatienten durch Wiegen oft nur schwer zu ermitteln.

Zwar ist eine bedarfsgerechte Ernährung adipöser Intensivpatienten mit Einzellösungen, bei denen über getrennte Behältnisse Aminosäuren, Kohlenhydrate und Lipide zugeführt werden, denkbar. Dies erfordert aber komplexe Berechnungen der benötigten Volumina an Ernährungslösungen durch den verschreibenden Arzt sowie die Applikation aus mehreren verschiedenen Behältnissen, was mit einem erhöhten Zeitaufwand des Pflegepersonals, einem erhöhten Risiko für das Auftreten von Verabreichungs- und Dosierungsfehlern sowie einem erhöhten Verbrauch an Ernährungslösungen und damit verbunden auch mit erhöhten Kosten einhergeht, da die in den Einzelbehältnissen enthaltenen Volumina nicht aufeinander abgestimmt sind und Restvolumina der einzelnen Lösungen verworfen werden müssen.

Angesichts dieser Nachteile parenteraler Ernährungslösungen des Stands der Technik soll eine parenterale Ernährungslösung zur Verabreichung an adipöse Intensivpatienten bereitgestellt werden, die die besonderen ernährungsphysiologischen Bedürfnisse dieser Patientengruppe berücksichtigt, und zugleich einfach, sicher und wirtschaftlich in Handhabung und Dosierbarkeit ist.

## Offenbarung der Erfindung

Die Erfindung umfasst eine parenterale Ernährungslösung zur Verwendung in der Verabreichung an einen adipösen Intensivpatienten, wobei die Ernährungslösung pro 1000 mL umfasst:

(i) 500 mL - 834 mL einer Aminosäurelösung umfassend 50 g - 83 g Aminosäuren,

(ii) 167 mL - 278 mL einer Glukoselösung umfassend 67 g - 111 g Glukose, und

(iii) 83 mL - 139 mL einer Lipidemulsion umfassend 17 g - 28 g Lipide.

Die parenterale Ernährungslösung kann pro 1000 mL umfassen:

(i) 667 mL einer Aminosäurelösung umfassend 67 g Aminosäuren,
(ii) 222 mL einer Glukoselösung umfassend 89 g Glukose, und

(iii) 111 mL einer Lipidemulsion umfassend 22 g Lipide.

In einer bevorzugten Ausführungsform umfasst die von der parenteralen Ernährungslösung umfasste Aminosäurelösung pro 1000 mL der Aminosäurelösung mindestens einen Bestandteil, der ausgewählt ist aus der Gruppe bestehend aus etwa 5 g Isoleucin, etwa 7,4 g Leucin, etwa 9,31 g Lysinacetat (entsprechend etwa 6,6 g Lysin), etwa 4,3 g Methionin, etwa 5,1 g Phenylalanin, etwa 4,4 g Threonin, etwa 2 g Tryptophan, etwa 6,2 g Valin, etwa 12 g Arginin, etwa 3 g Histidin, etwa 14 g Alanin, etwa 11 g Glycin, etwa 11,2 g Prolin, etwa 6,5 g Serin, etwa 0,4 g Tyrosin und etwa 1 g Taurin oder einer Kombination davon.

Ebenfalls bevorzugt ist es, dass die von der parenteralen Ernährungslösung umfasste Lipidemulsion pro 1000 mL der Lipidemulsion mindestens einen Bestandteil umfasst, der ausgewählt ist aus der Gruppe bestehend aus etwa 60 g Sojaöl, etwa 60 g MCT, etwa 50 g Olivenöl und etwa 30 g Fischöl oder einer Kombination davon.

Die parenterale Ernährungslösung kann ferner Elektrolyte umfassen. In einer bevorzugten Ausführungsform umfasst die parenterale Ernährungslösung pro 1000 mL Elektrolyte, die ausgewählt sind aus der Gruppe bestehend aus etwa 32,8 mmol Natrium bis etwa 48 mmol Natrium, etwa 24 mmol Kalium bis etwa 36 mmol Kalium, etwa 4,1 mmol Magnesium bis etwa 6,1 mmol Magnesium, etwa 2 mmol Calcium bis etwa 3 mmol Calcium, etwa 8,2 mmol Phosphat bis etwa 15,6 mmol Phosphat, etwa 0,032 mmol Zink bis etwa 0,048 mmol Zink, etwa 4,1 mmol Sulfat bis etwa 6,1 mmol Sulfat, etwa 28,8 mmol Chlorid bis etwa 43,2 mmol Chlorid, etwa 84,8 mmol Acetat bis etwa 127,2 mmol Acetat oder einer Kombination davon.

Eine besonders bevorzugte parenterale Ernährungslösung umfasst pro 1000 mL Elektrolyte, die ausgewählt sind aus der Gruppe bestehend aus etwa 36,9 mmol Natrium bis etwa 45,1 mmol Natrium, etwa 27 mmol Kalium bis etwa 33 mmol Kalium, etwa 4,6 mmol Magnesium bis etwa 5,6 mmol Magnesium, etwa 2,3 mmol Calcium bis etwa 2,8 mmol Calcium, etwa 9,2 mmol Phosphat bis etwa 14,3 mmol Phosphat, etwa 0,036 mmol Zink bis etwa 0,044 mmol Zink, etwa 4,6 mmol Sulfat bis etwa 5,6 mmol Sulfat, etwa 32,4 mmol Chlorid bis etwa 39,6 mmol Chlorid, etwa 95,4 mmol Acetat bis etwa 116,6 mmol Acetat oder einer Kombination davon.

Eine ganz besonders bevorzugte parenterale Ernährungslösung umfasst pro 1000 mL Elektrolyte, die ausgewählt sind aus der Gruppe bestehend aus etwa 36,9 mmol Natrium bis etwa 45,1 mmol Natrium, etwa 27 mmol Kalium bis etwa 33 mmol Kalium, etwa 4,6 mmol Magnesium bis etwa 5,6 mmol Magnesium, etwa 2,3 mmol Calcium bis etwa 2,8 mmol Calcium, etwa 10,2 mmol Phosphat bis etwa 13 mmol Phosphat, etwa 0,036 mmol Zink bis etwa 0,044 mmol Zink, etwa 4,6 mmol Sulfat bis etwa 5,6 mmol Sulfat, etwa 32,4 mmol Chlorid bis etwa 39,6 mmol Chlorid, etwa 95,4 mmol Acetat bis etwa 116,6 mmol Acetat oder einer Kombination davon.

Noch stärker bevorzugt ist, dass die parenterale Ernährungslösung pro 1000 mL Elektrolyte, die ausgewählt sind aus der Gruppe bestehend aus etwa 41 mmol Natrium, etwa 30 mmol Kalium, etwa 5,1 mmol Magnesium, etwa 2,5 mmol Calcium, etwa 13 mmol Phosphat, etwa 0,04 mmol Zink, etwa 5,1 mmol Sulfat, etwa 36 mmol Chlorid, etwa 106 mmol Acetat oder einer Kombination davon, umfasst.

Am meisten bevorzugt ist eine parenterale Ernährungslösung, die pro 1000 mL der Ernährungslösung 41 mmol Natrium, 30 mmol Kalium, 5,1 mmol Magnesium, 2,5 mmol Calcium, 13 mmol Phosphat, 0,04 mmol Zink, 5,1 mmol Sulfat, 36 mmol Chlorid und 106 mmol Acetat umfasst.

Die parenterale Ernährungslösung kann pro 100 Energieprozent 25 bis 35 Energieprozent aus Aminosäuren, 40 bis 45 Energieprozent aus Glucose und 22 bis 30 Energieprozent aus Lipiden umfassen. Bevorzugt umfasst die Ernährungslösung pro 100 Energieprozent 28 bis 34 Energieprozent aus Aminosäuren, 41 bis 44 Energieprozent aus Glucose und 25 bis 27 Energieprozent aus Lipiden. Besonders bevorzugt umfasst die Ernährungslösung pro 100 Energieprozent 32 Energieprozent aus Aminosäuren, 42 Energieprozent aus Glucose und 26 Energieprozent aus Lipiden.

Die parenterale Ernährungslösung weist einen Energiegehalt von 700 kcal bis 975 kcal pro 1000 mL auf. Bevorzugt ist, dass die Ernährungslösung einen Energiegehalt von 800 kcal bis 880 kcal pro 1000 mL aufweist. Am meisten bevorzugt ist eine Ernährungslösung, die einen Energiegehalt von 820 kcal bis 860 kcal pro 1000 mL aufweist.

Die parenterale Ernährungslösung kann in einem Dreikammerbeutel enthalten sein, der eine erste Kammer, eine zweite Kammer und eine dritte Kammer umfasst. Dabei befindet sich

(i) in der ersten Kammer die Aminosäurelösung,
(ii) in der zweiten Kammer die Glukoselösung,
und
(iii) in der dritten Kammer die Lipidemulsion,

wobei die Aminosäurelösung, die Glukoselösung und die Lipidemulsion vor der Verwendung zur Verabreichung an einen adipösen Intensivpatienten vereinigt und gemischt werden.

Umfasst die in dem Dreikammerbeutel enthaltene parenterale Ernährungslösung Elektrolyte, können diese in der ersten und/oder in der zweiten Kammer des Dreikammerbeutels enthalten sein.

Die Erfindung umfasst ferner einen Dreikammerbeutel umfassend eine erste Kammer, eine zweite Kammer und eine dritte Kammer, wobei

die erste Kammer 750 mL - 2250 mL einer Aminosäurelösung umfasst, enthaltend 56,3 g - 281,3 g Aminosäuren,
die zweite Kammer 250 mL - 750 mL einer Glukoselösung umfasst, enthaltend 75 g - 375 g Glukose,
und
die dritte Kammer 125 mL - 375 mL einer Lipidemulsion umfasst, enthaltend 18,8 g - 93,8 g Lipide,

wobei der Dreikammerbeutel, bezogen auf das Gesamtvolumen der ersten, der zweiten und der dritten Kammer, pro 1000 mL mindestens 50 g Aminosäuren umfasst, und wobei die in der parenteralen Ernährungslösung umfasste Lipidemulsion pro 1000 mL der Lipidemulsion mindestens einen Bestandteil, der ausgewählt ist aus der Gruppe bestehend aus: etwa 60 g Sojaöl, etwa 60 g MCT, etwa 50 g Olivenöl und etwa 30 g Fischöl oder einer Kombination davon umfasst.

Bevorzugt umfasst der Dreikammerbeutel, bezogen auf das Gesamtvolumen der ersten, der zweiten und der dritten Kammer, pro 1000 mL mindestens 60 g Aminosäuren, besonders bevorzugt mindestens 63 g Aminosäuren, noch stärker bevorzugt mindestens 65 g Aminosäuren.

In einer bevorzugten Ausführungsform umfasst der Dreikammerbeutel eine erste, eine zweite und eine dritte Kammer, wobei

die erste Kammer 1500 mL einer Aminosäurelösung enthaltend 135 g - 187,5 g Aminosäuren, bevorzugt 150 g Aminosäuren, umfasst,
die zweite Kammer 500 mL einer Glukoselösung enthaltend 150 g - 250 g Glucose, bevorzugt 200 g Glucose, umfasst, und
die dritte Kammer 250 mL einer Lipidemulsion enthaltend 37,5 g - 62,5 g Lipide, bevorzugt 50 g Lipide, umfasst, oder wobei
die erste Kammer 750 mL einer Aminosäurelösung enthaltend 67,5 g - 93,8 g Aminosäuren, bevorzugt 75 g Aminosäuren, umfasst,
die zweite Kammer 250 mL einer Glukoselösung enthaltend 75 g - 125 g Glucose, bevorzugt 100 g Glucose, umfasst, und die dritte Kammer 125 mL einer Lipidemulsion enthaltend 18,8 g - 31,3 g Lipide, bevorzugt 25 g Lipide, umfasst, oder wobei
die erste Kammer 2250 mL einer Aminosäurelösung enthaltend 202,5 g - 281,3 g Aminosäuren, bevorzugt 225 g Aminosäuren, umfasst,
die zweite Kammer 750 mL einer Glukoselösung enthaltend 225 g - 375 g Glucose, bevorzugt 300 g Glucose umfasst, und die dritte Kammer 375 mL einer Lipidemulsion enthaltend 56,3 g - 93,8 g Lipide, bevorzugt 75 g Lipide, umfasst.

Die parenterale Ernährungslösung ist zur Verabreichung an einen adipösen Intensivpatienten geeignet. In einer bevorzugten Ausführungsform wird die parenterale Ernährungslösung an einen adipösen Intensivpatienten mit einer hyperkatabolen und/oder hypermetabolen Stoffwechsellage verabreicht. In einer besonders bevorzugten Ausführungsform wird die parenterale Ernährungslösung an einen adipösen Intensivpatienten verabreicht, der kritisch krank ist und/oder der eine aus der Gruppe bestehend aus Trauma, Verbrennung, Infektion, Sepsis und chirurgischer Eingriff oder einer Kombination davon ausgewählte Erkrankung hat.

Die parenterale Ernährungslösung kann an einen adipösen Intensivpatienten mit

Adipositas Grad I, Adipositas Grad II und Adipositas Grad III verabreicht werden.

In einer weiteren bevorzugten Ausführungsform wird die täglich zu verabreichende Menge der parenteralen Ernährungslösung durch einen Parameter bestimmt, der ausgewählt ist aus der Gruppe bestehend aus Körpergröße des adipösen Intensivpatienten, Geschlecht des adipösen Intensivpatienten, Grad der Adipositas des adipösen Intensivpatienten oder einer Kombination davon.

Die parenterale Ernährungslösung wird, wenn der Patient an Adipositas Grad I oder Grad II leidet, dem Patienten in einer Menge von 28 mL bis 32 mL pro kg IBW und Tag verabreicht, besonders bevorzugt in einer Menge von 30 mL pro kg IBW und Tag.

Wenn der Patient an Adipositas Grad III leidet, wird die parenterale Ernährungslösung dem Patienten in einer Menge von 35 mL bis 39 mL pro kg IBW und Tag verabreicht, besonders bevorzugt in einer Menge von 37 mL pro kg IBW und Tag.

Wenn der Patient weiblich ist und an Adipositas Grad I oder Grad II leidet, können dem Patienten pro Tag etwa 2100 mL der parenteralen Ernährungslösung bei einer Körpergröße von 180 cm, etwa 1950 mL bei einer Körpergröße von 174 cm, etwa 1800 mL bei einer Körpergröße von 168 cm, etwa 1650 mL bei einer Körpergröße von 163 cm oder etwa 1500 mL bei einer Körpergröße von 157 cm verabreicht werden. Wenn der Patient weiblich ist und an Adipositas Grad III leidet, können dem Patienten pro Tag etwa 2590 mL der parenteralen Ernährungslösung bei einer Körpergröße von 180 cm, etwa 2405 mL bei einer Körpergröße von 174 cm, etwa 2220 mL bei einer Körpergröße von 168 cm, etwa 2035 mL bei einer Körpergröße von 163 cm oder etwa 1850 mL bei einer Körpergröße von 157 cm verabreicht werden.

Wenn der Patient männlich ist und an Adipositas Grad I oder Grad II leidet, können dem Patienten pro Tag etwa 2550 mL der parenteralen Ernährungslösung bei einer Körpergröße von 187 cm, etwa 2400 mL bei einer Körpergröße von 182 cm, etwa 2250 mL bei einer Körpergröße von 178 cm, etwa 2100 mL bei einer Körpergröße von 173 cm, etwa 1950 mL bei einer Körpergröße von 168 cm oder etwa 1800 mL bei einer Körpergröße von 163 cm verabreicht werden. Wenn der Patient männlich ist und an Adipositas Grad III leidet, können dem Patienten pro Tag etwa 3145 mL der parenteralen Ernährungslösung bei einer Körpergröße von 187 cm, etwa 2960 mL bei einer Körpergröße von 182 cm, etwa 2775 mL bei einer Körpergröße von 178 cm, etwa 2590 mL bei einer Körpergröße von 173 cm, etwa 2405 mL bei einer Körpergröße von 168 cm oder etwa 2220 mL bei einer Körpergröße von 163 cm verabreicht werden.

Weitere Ausführungsformen der Erfindung sind aus der folgenden eingehenden Beschreibung ersichtlich.

## Definitionen

Als **parenterale Ernährung** wird die Verabreichung von Nahrung (beispielsweise von geeigneten Ernährungslösungen) in die Blutbahn, d. h. unter Umgehung des Magen-Darm Traktes bezeichnet. Umfasst sind somit zum Beispiel die intravenöse Injektion oder Infusion sowie die intraarterielle Injektion oder Infusion. Bevorzugt wird die parenterale Ernährungslösung durch einen zentralen Venenkatheter verabreicht.

Als **intraarteriell** wird die Verabreichung in ein arterielles Blutgefäß verstanden, als **intravenös** die Verabreichung in ein venöses Blutgefäß. Mit **Injektion** wird die Verabreichung per Spritze bezeichnet. In der Regel erfolgt diese als Bolusgabe. Eine kontinuierliche Injektion mittels Spritzenpumpen ist aber ebenso möglich. Mit **Infusion** wird die kontinuierliche Verabreichung der Zusammensetzung in ein Blutgefäß bezeichnet, die zum Beispiel über einen peripheren oder zentralen Venenkatheter erfolgen kann.

Eine **parenterale Ernährungslösung** ist eine Ernährungslösung, die zur parenteralen Ernährung geeignet ist. Eine parenterale Ernährungslösung kann aufgrund ihrer Beschaffenheit in die Blutbahn eines Patienten appliziert werden.

**Adipositas** liegt bei starkem Übergewicht vor, das durch eine über das normale Maß hinausgehende Vermehrung des Körperfettes mit krankhaften Auswirkungen gekennzeichnet ist. Laut Definition der Word Health Organisation (WHO (Herausgeber): Obesity: preventing and managing the global epidemic. Report of a WHO Consultation. In: WHO Technical Report Series. 894, 2000) wird anhand des Körpermasseindex (body mass index; BMI) ermittelt, ob eine Adipositas vorliegt. Eine Adipositas liegt ab einem Körpermasseindex (BMI) von 30 kg/m$^2$ vor. Es werden drei Schweregrade der Adipositas unterschieden (Adipositas Grad I, Grad II oder Grad III), deren Unterteilung ebenfalls mittels des BMI erfolgt.

Eine **Adipositas Grad I** liegt bei einem BMI von 30,0 - 34,99 vor. Eine **Adipositas Grad II** liegt bei einem BMI von 35,0 - 39,99 vor. Eine **Adipositas Grad III** liegt ab einem BMI von ≥ 40 vor.

Der **Körpermasseindex (body mass index; BMI)** ist ein Maß zur Bewertung des Körpergewichts eines Menschen in Relation zu seiner Körpergröße, und wird nach der folgenden Formel berechnet: BMI = m/I$^2$

Dabei bezeichnet m die Körpermasse in kg und I die Körpergröße in m.

Das **Idealgewicht (ideal body weight, IBW)** in kg für Männer und Frauen kann nach den folgenden Formeln berechnet werden:

Für Männer :

$$\text{IBW} = 48{,}0 + (\text{Körpergröße} - 152) \times 1{,}06$$

Für Frauen:

$$\text{IBW} = 45{,}4 + (\text{Körpergröße} - 152) \times 0{,}89$$

Bei der Berechnung ist die Körpergröße in cm zu verwenden.

Ein **Intensivpatient** ist ein männlicher oder weiblicher Patient, der sich in stationärer, intensivmedizinischer ärztlicher Behandlung befindet. Ein "Intensivpatient" im Sinne der hier vorliegenden Anmeldung kann ein Patient sein, bei dem eine medizinische Indikation zur parenteralen Ernährung besteht. Eine medizinische Indikation zur parenteralen Ernährung kann bestehen, wenn eine orale oder enterale Ernährung nicht oder nicht in ausreichendem Ausmaß erfolgen kann, oder wenn eine orale oder enterale Ernährung kontraindiziert ist.

Der Ausdruck **"hyperkatabole Stoffwechsellage"** beschreibt einen überhöhten Abbaustoffwechsel.

Unter einer **"hypermetabolen Stoffwechsellage"** wir eine Steigerung des Stoffwechsels im gesamten Organismus verstanden.

Der Ausdruck **"kritisch krank"** umfasst Intensivpatienten, die an einem systemischen inflammatorischen Responsesyndrom (SIRS), an Sepsis oder an einem Multiorgandysfunktionssyndrom (MODS) leiden oder bei denen ein Risiko besteht, SIRS, MODS oder eine Sepsis zu entwickeln.

**Systemisches inflammatorisches Responsesyndrom (SIRS)** ist die Bezeichnung für eine generalisierte systemische Entzündungsreaktion auf ein schweres körperliches Ereignis (z.B. auf ein Trauma). SIRS ist in Bezug auf Sepsis der übergeordnete Begriff, da er das klinische Syndrom des kritisch kranken Patienten (unabhängig von der Ursache) beschreibt. Als nichtinfektiöse Auslöser kommen beispielsweise Pankreatitis, Ischämie, Trauma und Gewebeverletzungen, hämorrhagischer Schock oder Verbrennungen in Frage. Wird das SIRS durch eine nachgewiesene Infektion verursacht, wird es als **Sepsis** bezeichnet. Als Komplikation und Folge von SIRS und schwerer Sepsis tritt häufig ein Multiorganversagen auf. Ein Multiorgandysfunktionssyndrom (MODS) kann infolge von Schock, Sepsis oder SIRS auftreten, und ist durch Einschränkungen der Organfunktion, Organfehlfunktionen und/oder Organausfälle gekennzeichnet. Die Zahl der betroffenen Organe und die Schwere der auftretenden Organbeeinträchtigungen können dabei variieren.

Der Ausdruck **"etwa"** wie hier im Bezug auf Zahlenwerte verwendet bezeichnet eine Abweichung vom genannten Zahlenwert deren Größe unter anderem durch die Genauigkeit der Bestimmungsmethode des Zahlenwerts bestimmt wird. Bevorzugter Weise bezeichnet "etwa" in der vorliegenden Erfindung eine Abweichung von $\pm$ 5% des genannten Zahlenwertes.

Als **Energieprozent** eines Bestandteils der Ernährungslösung wird der Anteil des Bestandteils am Gesamtenergiegehalt der Ernährungslösung in Prozent bezeichnet. Der Gesamtenergiegehalt der Ernährungslösung beträgt 100%. Als der **Energiegehalt** einer Nahrung wird die Energiemenge bezeichnet, die der Körper beim Abbau einer Nahrung gewinnen kann. Die Angabe des Energiegehalts erfolgt in Kalorien (kcal) oder Joule (J).

Unter einem **Dipeptid** wird eine Verbindung verstanden, die aus zwei Aminosäuren besteht, welche über eine Peptidbindung miteinander verknüpft sind. Das Dipeptid kann N(2)-L-Alanyl-L-Glutamin sein.

Unter **Fischöl** wird aus Fisch gewonnenes Öl verstanden, das Omega-3-Fettsäuren enthält. Das Fischöl kann aus Seefischen, zum Beispiel aus Hochseefischen gewonnen werden. Bei **Fischöl** im Sinne der vorliegenden Anmeldung handelt es sich um hochaufgereinigtes Fischöl, das zur parenteralen Verabreichung beim Menschen geeignet ist.

**Mittelkettige Triglyceride (medium chain triglycerides, MCT)** sind Triglyceride, die Fettsäurereste mittlerer Länge (einer Länge von 6 bis 12 C-Atomen) umfassen. Beispiele für Fettsäuren von MCT sind Capronsäure ($C_6$), Caprylsäure ($C_8$), Caprinsäure ($C_{10}$), und Laurinsäure ($C_{12}$), oder einer Kombination davon.

Zu den **Spurenelementen** gehören Chrom (Cr) Cobalt (Co) Iod (I) Eisen (Fe) Kupfer (Cu) Mangan (Mn) Molybdän (Mo) Selen (Se) und Zink (Zn).

Gelöste, in positiv und negativ geladene Ionen dissoziierte Salze können auch als **Elektrolyte** bezeichnet werden. Zu den Elektrolyten zählen beispielsweise Natrium, Kalium, Magnesium, Calcium, Phosphat, Zink, Sulfat, Chlorid und Acetat.

Zu den **Vitaminen** gehören Vitamin A, Vitamin $B_1$, Vitamin $B_2$, Vitamin $B_3$, Vitamin $B_5$, Vitamin $B_6$, Vitamin $B_7$, Vitamin $B_9$, Vitamin $B_{12}$, Vitamin C, Vitamin D, Vitamin E und Vitamin K.

## Ausführungsformen der Erfindung

Die im Folgenden verwendeten Überschriften dienen ausschließlich der besseren Übersicht und sind nicht als limitierend aufzufassen. Werden im Folgenden Bereiche von Werten genannt, so ist jeder der durch diesen Bereich umfassten Einzelwerte ebenfalls offenbart.

## Zusammensetzung der parenteralen Ernährunaslösuna

Die parenterale Ernährungslösung ist aufgrund ihrer speziellen Zusammensetzung besonders gut zur parenteralen Ernährung von adipösen Intensivpatienten geeignet.

Die parenterale Ernährungslösung umfasst nur solche Bestandteile, die zur parenteralen Verabreichung beim Menschen geeignet sind. Alle verwendeten Bestandteile weisen einen Reinheitsgrad auf, bei dem sich der jeweilige Bestandteil zur parenteralen Verabreichung eignet. Dem Fachmann sind zur parenteralen Verabreichung an Menschen geeignete Bestandteile sowie deren benötigter Reinheitsgrad bekannt.

Die parenterale Ernährungslösung setzt sich aus einer Aminosäurelösung, einer Glukoselösung und eine Lipidemulsion zusammen, und kann ferner Elektrolyte umfassen.

Umfasst die parenterale Ernährungslösung Elektrolyte, so können diese in der Aminosäurelösung und/oder in der Glucoselösung enthalten sein.

In der parenteralen Ernährungslösung können die Aminosäurelösung, die Glukoselösung und die Lipidemulsion in vereinigter und gemischter Form vorliegen.

Vor der Verabreichung an einen adipösen Intensivpatienten können der parenteralen Ernährungslösung Spurenelemente und/oder Vitamine zugesetzt werden. Ebenso können vor der Verabreichung Elektrolyte zugesetzt werden, wenn diese nicht bereits in der Aminosäurelösung und/oder der Glukoselösung enthalten sind oder wenn eine zusätzliche Gabe von Elektrolyten ergänzend zu bereits in der parenteralen Ernährungslösung enthaltenen Elektrolyten gewünscht ist.

Alternativ oder ergänzend können Elektrolyte, Spurenelemente und/oder Vitamine auch getrennt von der parenteralen Ernährungslösung verabreicht werden.

### (i) Aminosäurelösung

Der Aminosäurebedarf von adipösen Intensivpatienten ist im Vergleich zu normalgewichtigen Intensivpatienten erhöht, und beträgt mindestens 2 g/kg Körpergewicht (bezogen auf das Idealgewicht des Patienten) und Tag für Intensivpatienten mit Adipositas Grad I oder II. Bei Intensivpatienten mit Adipositas Grad III ist der Aminosäurebedarf mit mindestens 2,5 g/kg Körpergewicht (bezogen auf das Idealgewicht des Patienten) und Tag noch höher anzusetzen.

Eine ausreichende Versorgung mit Aminosäuren ist beispielsweise wichtig für den Erhalt der fettfreien Körpermasse, insbesondere der Muskelmasse des Patienten. Besonders bei Intensivpatienten mit Verletzungen, Verbrennungen oder nach einem chirurgischen Eingriff ist eine ausreichende Versorgung mit Aminosäuren auch für die Wundheilung von großer Bedeutung. Es ist daher entscheidend, dass dem adipösen Intensivpatienten mit der parenteralen Ernährungslösung Aminosäuren in ausreichender Menge verabreicht werden.

Die Erfinder haben in diesem Zusammenhang herausgefunden, dass eine parenterale Ernährungslösung, die pro mL 0,050 g bis 0,083 g Aminosäuren umfasst, gut geeignet zur Verabreichung an adipöse Intensivpatienten ist, da so einerseits der erhöhte Aminosäurebedarf der adipösen Intensivpatienten gedeckt werden kann, es andererseits aber nicht zu einem übermäßigen Anstieg von Endprodukten des Proteinstoffwechsels kommt.

Überraschend hat sich dabei gezeigt, dass eine parenterale Ernährungslösung, die pro mL 0,060 g bis 0,083 g Aminosäuren, insbesondere 0,063 g bis 0,080 g Aminosäuren, umfasst, zur parenteralen Ernährung adipöser Intensivpatienten besonders gut geeignet ist. Bevorzugt ist eine parenterale Ernährungslösung, die pro mL 0,065 g bis 0,075 g Aminosäuren umfasst. Besonders bevorzugt ist eine parenterale Ernährungslösung, die pro mL 0,065 g bis 0,070 g Aminosäuren umfasst. Am meisten bevorzugt ist eine parenterale Ernährungslösung, die pro mL 0,067 g Aminosäuren umfasst.

In einer besonders bevorzugten Ausführungsform umfasst die in der parenteralen Ernährungslösung enthaltene Aminosäurelösung pro 1000 mL der Aminosäurelösung mindestens einen Bestandteil, der ausgewählt ist aus der Gruppe bestehend aus: etwa 5 g Isoleucin, etwa 7,4 g Leucin, etwa 9,31 g Lysinacetat (entsprechend etwa 6,6 g Lysin), etwa 4,3 g Methionin, etwa 5,1 g Phenylalanin, etwa 4,4 g Threonin, etwa 2 g Tryptophan, etwa 6,2 g Valin, etwa 12 g Arginin, etwa 3 g Histidin, etwa 14 g Alanin, etwa 11 g Glycin, etwa 11,2 g Prolin, etwa 6,5 g Serin, etwa 0,4 g Tyrosin und etwa 1 g Taurin oder einer Kombination davon.

Ganz besonders bevorzugt ist eine Ausführungsform, in der die in der parenteralen Ernährungslösung enthaltene Aminosäurelösung pro 1000 mL der Aminosäurelösung 5 g Isoleucin, 7,4 g Leucin, 9,31 g Lysinacetat (entsprechend 6,6 g Lysin), 4,3 g Methionin, 5,1 g Phenylalanin, 4,4 g Threonin, 2 g Tryptophan, 6,2 g Valin, 12 g Arginin, 3 g Histidin, 14 g Alanin, 11 g Glycin, 11,2 g Prolin, 6,5 g Serin, 0,4 g Tyrosin und 1 g Taurin umfasst.

Der parenteralen Ernährungslösung können in einer weiteren bevorzugten Ausführungsform vor der Verabreichung an einen adipösen Intensivpatienten, zusätzlich zu den in der parenteralen Ernährungslösung enthaltenen Aminosäuren, Aminosäuren in Form von Dipeptiden zugesetzt werden. Der parenteralen Ernährungslösung können pro mL 0,01 g bis 0,04 g Dipeptid, bevorzugt 0,015 g bis 0,030 g Dipeptid, am meisten bevorzugt 0,02 g bis 0,025 g Dipeptid zugesetzt werden. In einer besonders bevorzugten Ausführungsform ist das Dipeptid N(2)-L-Alanyl-L-Glutamin.

### (ii) Glukoselösung

Die parenterale Ernährungslösung zeichnet sich durch einen vergleichsweise geringen Glukosegehalt aus, der niedriger ist als der von aus dem Stand der Technik bekannten aminosäurereichen parenteralen Ernährungslösungen.

In der parenteralen Ernährungslösung zur Verabreichung an adipöse Intensivpatienten werden Kohlenhydrate in Form von Glukose bereitgestellt. Die parenterale Ernährungslösung umfasst pro mL 0,067 g bis 0,111 g Glukose.

Bevorzugt umfasste die parenterale Ernährungslösung umfasst pro mL 0,070 g bis 0,105 g Glukose, oder 0,080 g bis 0,095 g Glukose. Besonders bevorzugt ist eine parenterale Ernährungslösung, die pro mL 0,082 g bis 0,093 g Glukose umfasst. Am meisten bevorzugt ist eine parenterale Ernährungslösung, die pro mL 0,089 g Glukose umfasst.

Zwar ist die Verabreichung von Kohlenhydraten, zum Beispiel in Form von Glukose, für eine bedarfsdeckende parenterale Ernährung auch bei adipösen Intensivpatienten unerlässlich. Allerdings ist der vergleichsweise geringe Glukosegehalt der vorliegenden parenteralen Sondennahrung äußerst vorteilhaft bei der Verabreichung an adipöse Intensivpatienten, da in dieser Patientengruppe sehr häufig bereits eine Störung des Glukosestoffwechsels in Form einer Glukoseintoleranz oder eines manifesten Diabetes mellitus vorliegt oder während der parenteralen Ernährung auftritt.

**(iii) Lipidemulsion**

Neben abweichenden Bedürfnissen bei der Versorgung mit Aminosäuren und Kohlenhydraten bei der parenteralen Ernährung unterscheiden sich adipöse Intensivpatienten von normal- oder untergewichtigen Intensivpatienten auch im Lipidbedarf, denn der Lipidstoffwechsel ist, ähnlich wie der Kohlenhydratstoffwechsel, bei vielen adipösen Intensivpatienten bereits vor dem Auftreten des Ereignisses, das eine parenterale Ernährung des Patienten nötig macht, gestört. Zudem sind adipöse Intensivpatienten bei parenteraler Ernährung einem höheren Risiko als normal- oder untergewichtige Intensivpatienten ausgesetzt, durch eine inadäquate, nicht auf die besonderen Bedürfnisse von adipösen Intensivpatienten abgestimmte parenterale Ernährung eine Störung im Lipidstoffwechsel zu entwickeln. Zu den Lipidstoffwechselstörungen, die bei adipösen Intensivpatienten häufig bereits vor Begin der parenteralen Ernährung vorliegen und die, sofern sie nicht bereits vorliegen, häufig als Folge der parenteralen Ernährung auftreten, zählt beispielsweise die Hypertriglyceridämie. Mit der Hypertriglyceridämie einher geht häufig eine Erhöhung von Transaminasen, Bilirubin, Thrombozytopenie und Thrombozytopathie.

Durch die besondere Zusammensetzung der parenteralen Ernährungslösung und den vergleichsweise geringen durch Lipide zur Verfügung gestellten Energieanteil kann dem Auftreten von ernährungsbedingten Lipidstoffwechselstörungen in der Gruppe der adipösen Intensivpatienten in vorteilhafter Weise vorgebeugt werden. Liegt bereits eine Störung des Lipidstoffwechsels vor, kann einer Verstärkung der Stoffwechselstörung vorgebeugt und ein Entgleisen der Stoffwechselstörung aufgrund der parenteralen Ernährung vermieden werden.

Die parenterale Ernährungslösung umfasst pro mL 0,017 g bis 0,028 g Lipide. Bevorzugt ist dabei eine parenterale Ernährungslösung, die pro mL 0,019 g bis 0,026 g Lipide umfasst, oder die 0,020 g bis 0,024 g Lipide umfasst. Besonders bevorzugt umfasst die parenterale Ernährungslösung 0,021 g bis 0,023 g Lipide pro mL. Am meisten bevorzugt ist eine parenterale Ernährungslösung, die pro mL 0,022 g Lipide umfasst; diese hat sich als besonders wirksam erwiesen.

Als besonders vorteilhaft in der Ernährung adipöser Intensivpatienten hat sich eine parenterale Ernährungslösung erwiesen, bei der die umfassten Lipide eine bestimmte Zusammensetzung aufweisen. Die in der parenteralen Ernährungslösung umfasste Lipidemulsion umfasst pro 1000 mL der Lipidemulsion mindestens einen Bestandteil, der ausgewählt ist aus der Gruppe bestehend aus: etwa 60 g Sojaöl, etwa 60 g MCT, etwa 50 g Olivenöl und etwa 30 g Fischöl oder einer Kombination davon.

Ganz besonders bevorzugt ist es, dass die Lipidemulsion pro 1000 mL 60 g Sojaöl, 60 g MCT, 50 g Olivenöl und 30 g Fischöl umfasst.

Mit einer Lipidemulsion dieser Zusammensetzung kann das Auftreten oder die Verschlechterung einer bereits bestehenden entzündlichen Stoffwechsellage, die bei adipösen Intensivpatienten unabhängig von der Grunderkrankung oft vorliegt, verhindert werden.

**(iv) Energiegehalt und Energieprozent**

Parenterale Ernährungslösungen aus dem Stand der Technik sind unter anderem deshalb nicht auf die speziellen Ernährungsbedürfnisse adipöser Intensivpatienten ausgerichtet, weil sie einen vergleichsweise hohen Energiegehalt aufweisen. Dies erklärt sich unter anderem dadurch, dass die parenteralen Ernährungslösungen aus dem Stand der Technik im Allgemeinen auf den krankheitsbedingt erhöhten Energiebedarf normal- oder untergewichtiger Patienten zugeschnitten sind. Für diese Patientengruppe wird beispielsweise ein Energiebedarf von 25 kcal bis 30 kcal/kg tatsächliches Körpergewicht und Tag angesetzt.

Werden adipöse Intensivpatienten entsprechend ernährt, kommt es zu einer Überversorgung mit Energie, die aufgrund der Zusammensetzung der parenteralen Ernährungslösungen des Stands der Technik zudem überwiegend aus Kohlenhydraten und Lipiden stammt. Dadurch wird das Risiko für das Auftreten von ernährungsbedingten metabolischen und infektiösen Komplikationen in der Gruppe der adipösen Intensivpatienten erhöht.

Mit der parenteralen Ernährungslösung zur Verabreichung an adipöse Intensivpatienten wird nun eine Ernährungslösung bereitgestellt, die einen für die Ernährung von adipösen Intensivpatienten besonders geeigneten Energiegehalt aufweist.

Der Energiegehalt der parenteralen Ernährungslösung beträgt 700 kcal bis 950 kcal pro 1000 ml, bevorzugt ist ein Energiegehalt von 750 kcal bis 900 kcal pro 1000 ml, ebenso ein Energiegehalt von 800 kcal bis 880 kcal pro 1000 ml. Besonders bevorzugt ist ein Energiegehalt von 820 kcal bis 860 kcal pro 1000 ml, am meisten bevorzugt ist ein Energiegehalt von 845 kcal bis 855 kcal pro 1000 ml, wie etwa 850 kcal oder 851 kcal pro 1000 mL.

Die parenterale Ernährungslösung ist aufgrund ihrer Zusammensetzung im Bezug auf das Verhältnis von Aminosäuren, Glukose und Lipiden zur Ernährung von adipösen Intensivpatienten besonders gut geeignet.

Die parenterale Ernährungslösung kann pro 100 Energieprozent, also bezogen auf den Gesamtenergiegehalt der Ernährungslösung, 25 - 35 Energieprozent aus Aminosäuren, 40 - 45 Energieprozent aus Glucose und 22 - 30 Energieprozent aus Lipiden umfassen. Bevorzugt ist eine Ernährungslösung, die pro 100 Energieprozent 28 - 34 (28, 29, 30, 31, 32, 33, 34) Energieprozent aus Aminosäuren, 41 - 44 (41, 42, 43, 44) Energieprozent aus Glucose und 23 - 27 (23, 24, 25, 26, 27) Energieprozent aus Lipiden umfasst. Besonders bevorzugt ist eine Aminosäurelösung, die 31 - 33 (31, 32, 33) Energieprozent aus Aminosäuren, 42 - 44 (42, 43, 44) Energieprozent aus Glucose und 25 - 27 (25, 26, 27) Energieprozent aus Lipiden umfasst.

Am meisten bevorzugt ist eine parenterale Ernährungslösung, die 32 Energieprozent aus Aminosäuren, 42 Energieprozent aus Glukose und 26 Energieprozent aus Lipiden umfasst. Bei der Verabreichung einer parenteralen Ernährungslösung dieser Zusammensetzung an adipöse Intensivpatienten kommt es zu einer unerwartet geringen Rate metabolischer und infektiöser Komplikationen.

Durch den hohen Energieprozentanteil an Aminosäuren bei vergleichsweise geringen Anteilen an Glucose und Lipiden kann einer Über- oder Fehlernährung der adipösen Intensivpatienten und dem damit assoziierten Auftreten ernährungsbedingter metabolischer Komplikationen wie Hyperlipidämie, Hyperglycämie und Glucoseintoleranz wirksam vorgebeugt werden. Bei bereits vor Beginn der parenteralen Ernährung bestehenden, adipositasbedingten Stoffwechselstörungen kann eine Verstärkung der jeweiligen Stoffwechselstörung durch die besondere Zusammensetzung der parenteralen Ernährungslösung verhindert werden.

Auch kann durch die Zusammensetzung der parenteralen Ernährungslösung eine Unterversorgung der adipösen Intensivpatienten, insbesondere mit Aminosäuren, und das Auftreten von Protein-Energie-Malnutrition vermieden werden.

Zudem kann bei Verabreichung der parenteralen Ernährungslösung das Auftreten infektionsbedingter Komplikationen vermindert werden. In Folge kann auch die Morbiditäts- und Mortalitätsrate der adipösen Intensivpatienten verringert werden. Daneben kann auch eine Verkürzung der Aufenthaltsdauer auf der Intensivstation erreicht werden.

Durch die Zusammensetzung der parenteralen Ernährungslösung und deren hohen Aminosäureanteil wird die Mobilisierung des körpereigenen Fetts der adipösen Intensivpatienten erleichtert. Die parenterale Ernährungslösung ermöglicht somit eine optimale Mobilisierung der körpereigenen Energiereserven, durch den hohen Aminosäuregehalt gleichzeitig aber einen optimalen Erhalt körpereigener Proteine und Muskelmasse.

## Elektrolyte, Spurenelemente und Vitamine

In der parenteralen Ernährungslösung können Elektrolyte enthalten sein. Enthält die parenterale Ernährungslösung Elektrolyte, können diese in der Aminosäurelösung und/oder der Glukoselösung enthalten sein oder der parenteralen Ernährungslösung vor Verabreichung an den adipösen Intensivpatienten zugesetzt werden. Die Zugabe kann in dem Fachmann bekannter Weise, beispielsweise über einen Zuspritzport erfolgen.

In einer bevorzugten Ausführungsform umfasst die parenterale Ernährungslösung pro 1000 mL Elektrolyte, die ausgewählt sind aus der Gruppe bestehend aus etwa 32,8 mmol Natrium bis etwa 48 mmol Natrium, etwa 24 mmol Kalium bis etwa 36 mmol Kalium, etwa 4,1 mmol Magnesium bis etwa 6,1 mmol Magnesium, etwa 2 mmol Calcium bis etwa 3 mmol Calcium, etwa 8,2 mmol Phosphat bis etwa 15,6 mmol Phosphat, etwa 0,032 mmol Zink bis etwa 0,048 mmol Zink, etwa 4,1 mmol Sulfat bis etwa 6,1 mmol Sulfat, etwa 28,8 mmol Chlorid bis etwa 43,2 mmol Chlorid, etwa 84,8 mmol Acetat bis etwa 127,2 mmol Acetat oder einer Kombination davon.

Eine besonders bevorzugte parenterale Ernährungslösung umfasst pro 1000 mL Elektrolyte, die ausgewählt sind aus der Gruppe bestehend aus etwa 36,9 mmol Natrium bis etwa 45,1 mmol Natrium, etwa 27 mmol Kalium bis etwa 33 mmol Kalium, etwa 4,6 mmol Magnesium bis etwa 5,6 mmol Magnesium, etwa 2,3 mmol Calcium bis etwa 2,8 mmol Calcium, etwa 9,2 mmol Phosphat bis etwa 14,3 mmol Phosphat, etwa 0,036 mmol Zink bis etwa 0,044 mmol Zink, etwa 4,6 mmol Sulfat bis etwa 5,6 mmol Sulfat, etwa 32,4 mmol Chlorid bis etwa 39,6 mmol Chlorid, etwa 95,4 mmol Acetat bis etwa 116,6 mmol Acetat oder einer Kombination davon.

Eine ganz besonders bevorzugte parenterale Ernährungslösung umfasst pro 1000 mL Elektrolyte, die ausgewählt sind aus der Gruppe bestehend aus etwa 36,9 mmol Natrium bis etwa 45,1 mmol Natrium, etwa 27 mmol Kalium bis etwa 33 mmol Kalium, etwa 4,6 mmol Magnesium bis etwa 5,6 mmol Magnesium, etwa 2,3 mmol Calcium bis etwa 2,8 mmol Calcium, etwa 10,2 mmol Phosphat bis etwa 13 mmol Phosphat, etwa 0,036 mmol Zink bis etwa 0,044 mmol Zink, etwa 4,6 mmol Sulfat bis etwa 5,6 mmol Sulfat, etwa 32,4 mmol Chlorid bis etwa 39,6 mmol Chlorid, etwa 95,4 mmol Acetat bis etwa 116,6 mmol Acetat oder einer Kombination davon.

Noch stärker bevorzugt ist, dass die parenterale Ernährungslösung pro 1000 mL Elektrolyte, die ausgewählt sind aus der Gruppe bestehend aus etwa 41 mmol Natrium, etwa 30 mmol Kalium, etwa 5,1 mmol Magnesium, etwa 2,5 mmol Calcium, etwa 13 mmol Phosphat, etwa 0,04 mmol Zink, etwa 5,1 mmol Sulfat, etwa 36 mmol Chlorid, etwa 106 mmol Acetat oder einer Kombination davon, umfasst.

Am meisten bevorzugt ist eine parenterale Ernährungslösung, die pro 1000 mL der Ernährungslösung 41 mmol Natrium, 30 mmol Kalium, 5,1 mmol Magnesium, 2,5 mmol Calcium, 13 mmol Phosphat, 0,04 mmol Zink, 5,1 mmol Sulfat, 36 mmol Chlorid und 106 mmol Acetat umfasst.

Der parenteralen Ernährungslösung können Spurenelemente und/oder Vitamine vor der Verabreichung an den adipösen Intensivpatienten zugesetzt werden. Die Zugabe kann in dem Fachmann bekannter Weise, beispielsweise über einen Zuspritzport erfolgen.

Bevorzugt ist, dass vor der Verabreichung Spurenelemente und Vitamine in bedarfsdeckender Menge zugesetzt werden, so dass keine zusätzliche Verabreichung von Spurenelementen und Vitaminen an den adipösen Intensivpatienten mehr erforderlich ist.

Dabei kann bevorzugt mit der parenteralen Ernährungslösung täglich die doppelte Menge der für normalgewichtige Gesunde gleichen Alters und Geschlechts empfohlenen täglichen Menge an Spurenelementen und/oder Vitaminen verabreicht werden. Es hat sich herausgestellt, dass adipöse Intensivpatienten ganz besonders von einer parenteralen Ernährungslösung profitieren, über die eine solche, hohe Menge an Spurenelementen und/oder Vitaminen bereitgestellt wird, da gerade adipöse Intensivpatienten häufig unter multiplem Spurenelement- und/oder Vitaminmangel leiden.

**Bevorzugte Ausführungsformen**

Die parenterale Ernährungslösung zur Verabreichung an einen adipösen Intensivpatienten kann pro 1000 mL umfassen:

(i) 500 mL - 834 mL einer Aminosäurelösung umfassend 50 g - 83 g Aminosäuren,
(ii) 167 mL - 278 mL einer Glukoselösung umfassend 67 g - 111 g Glukose,
und
(iii) 83 mL - 139 mL einer Lipidemulsion umfassend 17 g - 28 g Lipide.

Somit umfasst die parenterale Ernährungslösung zur Verabreichung an einen adipösen Intensivpatienten pro mL 0,05 g bis 0,083 g Aminosäuren, 0,067 g bis 0,111 g Kohlenhydrate in Form von Glukose und 0,017 g bis 0,028 g Lipide.

Die parenterale Ernährungslösung zur Verabreichung an einen adipösen Intensivpatienten kann pro 1000 mL umfassen:

(i) 500 mL - 834 mL einer Aminosäurelösung umfassend 60 g - 83 g Aminosäuren,
(ii) 167 mL - 278 mL einer Glukoselösung umfassend 67 g - 111 g Glukose,
und
(iii) 83 mL - 139 mL einer Lipidemulsion umfassend 17 g - 28 g Lipide.

Somit umfasst die parenterale Ernährungslösung zur Verabreichung an einen adipösen Intensivpatienten pro mL 0,06 g bis 0,083 g Aminosäuren, 0,067 g bis 0,111 g Kohlenhydrate in Form von Glukose und 0,017 g bis 0,028 g Lipide.

Die parenterale Ernährungslösung kann pro mL 0,063 g bis 0,080 g Aminosäuren, 0,070 g bis 0,105 g Kohlenhydrate in Form von Glukose und 0,019 g bis 0,026 g Lipide umfassen.

Die parenterale Ernährungslösung zur Verabreichung an einen adipösen Intensivpatienten kann also beispielsweise pro 1000 mL umfassen:

(i) 500 mL - 834 mL einer Aminosäurelösung umfassend 63 g - 80 g Aminosäuren,
(ii) 167 mL - 278 mL einer Glukoselösung umfassend 70 g - 105 g Glukose,
und
(iii) 83 mL - 139 mL einer Lipidemulsion umfassend 19 g - 26 g Lipide.

Bevorzugt ist eine parenterale Ernährungslösung, die pro mL 0,065 g bis 0,075 g Aminosäuren, 0,080 g bis 0,095 g Kohlenhydrate in Form von Glukose und 0,020 g bis 0,024 g Lipide umfasst.

Dies ist beispielsweise bei der folgenden bevorzugten parenteralen Ernährungslösung der Fall, die pro 1000 mL umfasst:

(i) 500 mL - 834 mL einer Aminosäurelösung umfassend 65 g - 75 g Aminosäuren,

(ii) 167 mL - 278 mL einer Glukoselösung umfassend 80 g - 95 g Glukose, und
(iii) 83 mL - 139 mL einer Lipidemulsion umfassend 20 g - 24 g Lipide.

Noch stärker bevorzugt ist eine parenterale Ernährungslösung zur Verabreichung an einen adipösen Intensivpatienten, die pro 1000 mL umfasst:

(i) 500 mL - 834 mL einer Aminosäurelösung umfassend 65 g - 70 g Aminosäuren,
(ii) 167 mL - 278 mL einer Glukoselösung umfassend 82 g - 93 g Glukose, und
(iii) 83 mL - 139 mL einer Lipidemulsion umfassend 21 g - 23 g Lipide.

In dieser besonders bevorzugten Ausführungsform der parenteralen Ernährungslösung sind pro mL 0,065 g bis 0,070 g Aminosäuren, 0,082 g bis 0,093 g Kohlenhydrate in Form von Glukose und 0,021 g bis 0,023 g Lipide umfasst.

Ganz besonders bevorzugt ist eine parenterale Ernährungslösung, die pro 1000 mL umfasst:

(i) 667 mL einer Aminosäurelösung umfassend 67 g Aminosäuren,
(ii) 222 mL einer Glukoselösung umfassend 89 g Glukose, und
(iii) 111 mL einer Lipidemulsion umfassend 22 g Lipide.

In dieser ganz besonders bevorzugten Ausführungsform umfasst die parenterale Ernährungslösung zur Verabreichung an einen adipösen Intensivpatienten pro mL 0,067 g Aminosäuren, 0,089 g Kohlenhydrate in Form von Glukose und 0,022 g Lipide.

In jeder der vorstehend genannten Ausführungsformen kann die parenterale Ernährungslösung Elektrolyte umfassen. Diese können in der Aminosäurelösung und/oder der Glukoselösung enthalten sein.

### Dreikammerbeutel

Die parenterale Ernährungslösung kann in einem Dreikammerbeutel bereitgestellt werden. Dabei können die in der parenteralen Ernährungslösung umfasste Aminosäurelösung, die Glukoselösung und die Lipidemulsion jeweils getrennt von einander in einer eigenen Kammer des Dreikammerbeutels enthalten sein.

Jeder Dreikammerbeutel des Stands der Technik, der für die Aufbewahrung und Verabreichung parenteraler Ernährungslösungen geeignet ist, kann dabei verwendet werden.

Die einzelnen Kammern (also die erste Kammer, die zweite Kammer und die dritte Kammer) des Dreikammerbeutels können durch Peelnähte voneinander getrennt sein. Vor der Verabreichung der parenteralen Ernährungslösung werden die Peelnähte durch Druck geöffnet und der Inhalt der drei Kammern, die Aminosäurelösung, die Glukoselösung und die Lipidemulsion, miteinander vereinigt und gemischt.

In einer ersten Ausführungsform umfasst der Dreikammerbeutel eine erste Kammer, eine zweite Kammer und eine dritte Kammer, dabei umfasst die erste Kammer 750 mL - 2250 mL einer Aminosäurelösung umfassend 56,3 g - 281,3 g Aminosäuren, die zweite Kammer umfasst 250 mL - 750 mL einer Glukoselösung umfassend 75 g - 375 g Glukose, und die dritte Kammer umfasst 125 mL - 375 mL einer Lipidemulsion umfassend 18,8 g - 93,8 g Lipide.

Bezogen auf das Gesamtvolumen der ersten, der zweiten und der dritten Kammer, umfasst der Dreikammerbeutel pro 1000 mL mindestens 50 g Aminosäuren, beispielsweise 50 g bis 83 g Aminosäuren. Bevorzugt ist ein Dreikammerbeutel, der bezogen auf das Gesamtvolumen der ersten, der zweiten und der dritten Kammer, pro 1000 mL mindestens 60 g, beispielsweise 60 g bis 83 g, Aminosäuren umfasst. Besonders bevorzugt ist ein Dreikammerbeutel, der bezogen auf das Gesamtvolumen der ersten, der zweiten und der dritten Kammer pro 1000 mL mindestens 63 g, beispielsweise 63 g bis 83 g, Aminosäuren umfasst. Ganz besonders bevorzugt ist es, dass der Dreikammerbeutel bezogen auf das Gesamtvolumen der ersten, der zweiten und der dritten Kammer pro 1000 mL mindestens 65 g Aminosäuren, beispielsweise 65 g bis 83 g, umfasst. Am meisten bevorzugt ist dabei ein Dreikammerbeutel, der bezogen auf das Gesamtvolumen der ersten, der zweiten und der dritten Kammer pro 1000 mL mindestens 67 g Aminosäuren, beispielsweise 67 g bis 83 g, umfasst.

In einer weiteren, bevorzugten Ausführungsform umfasst der Dreikammerbeutel eine erste Kammer, eine zweite Kammer und eine dritte Kammer, wobei

die erste Kammer 1500 mL einer Aminosäurelösung enthaltend 135 g - 187,5 g Aminosäuren, bevorzugt 150 g Aminosäuren, umfasst,
die zweite Kammer 500 mL einer Glukoselösung enthaltend 150 g - 250 g Glucose, bevorzugt 200 g Glucose, umfasst, und
die dritte Kammer 250 mL einer Lipidemulsion enthaltend 37,5 g - 62,5 g Lipide, bevorzugt 50 g Lipide, umfasst.

Ebenfalls bevorzugt ist eine Ausführungsform, in der der Dreikammerbeutel eine erste Kammer, eine zweite Kammer und eine dritte Kammer umfasst, wobei

die erste Kammer 750 mL einer Aminosäurelösung enthaltend 67,5 g - 93,8 g Aminosäuren, bevorzugt 75 g Aminosäuren, umfasst,
die zweite Kammer 250 mL einer Glukoselösung enthaltend 75 g - 125 g Glucose, bevorzugt 100 g Glucose, umfasst, und die dritte Kammer 125 mL einer Lipidemulsion enthaltend 18,8 g - 31,3 g Lipide, bevorzugt 25 g Lipide, umfasst.

Auch bevorzugt ist eine Ausführungsform, in der der Dreikammerbeutel eine erste Kammer, eine zweite Kammer und eine dritte Kammer umfasst, wobei

die erste Kammer 2250 mL einer Aminosäurelösung enthaltend 202,5 g - 281,3 g Aminosäuren, bevorzugt 225 g Aminosäuren, umfasst,
die zweite Kammer 750 mL einer Glukoselösung enthaltend 225 g - 375 g Glucose, bevorzugt 300 g Glucose umfasst, und die dritte Kammer 375 mL einer Lipidemulsion enthaltend 56,3 g - 93,8 g Lipide, bevorzugt 75 g Lipide, umfasst.

In einer besonders bevorzugten Ausführungsform umfasst der Dreikammerbeutel ferner Elektrolyte. Elektrolyte können in der ersten und/oder in der zweiten Kammer des Dreikammerbeutels umfasst sein, also in der Aminosäurelösung und/oder in der Glukoselösung.
Spurenelemente und/oder Vitamine können dem Dreikammerbeutel vor der Verabreichung der parenteralen Ernährungslösung an einen adipösen Intensivpatienten zugesetzt werden. Bevorzugt werden Vitamine und Spurenelemente in bedarfsdeckender Menge zugesetzt. Enthalten die Aminosäurelösung und die Glukoselösung keine Elektrolyte, oder sollen Elektrolyte zusätzlich zu bereits in der Aminosäurelösung und/oder der Glukoselösung enthaltenen Elektrolyten verabreicht werden, können diese der parenteralen Ernährungslösung ebenfalls vor der Verabreichung zugesetzt werden. Die Zugabe erfolg in dem Fachmann bekannter Weise, beispielsweise über einen Zuspritzport.

## Vorteile

Die auf die besonderen Bedürfnisse adipöser Intensivpatienten abgestellte Zusammensetzung der parenteralen Ernährungslösung, insbesondere der hohe Aminosäurengehalt in Verbindung mit dem vergleichsweise geringen Gehalt an Glukose und Lipiden, kann in besonders vorteilhafter Weise überraschend zu einer Reduktion der Häufigkeit des Auftretens von durch die parenterale Ernährung bedingten ernährungsbedingten metabolischen sowie infektiösen Komplikationen, und zu einer Reduktion der Morbidität und Mortalität der adipösen Intensivpatienten führen.
Die parenterale Ernährungslösung ist kalorienarm, aber reich an Aminosäuren. Dadurch kann eine Überversorgung mit Kalorien vermieden, aber gleichzeitig einem übermäßigen Verlust an fettfreier Körpermasse, insbesondere Muskelmasse, des adipösen Intensivpatienten wirksam entgegengetreten werden.
Das Verhältnis, in dem Aminosäuren, Kohlenhydrate und Lipide in der parenteralen Ernährungslösung umfasst sind, wirkt sich besonders günstig bei der Vermeidung oder Verminderung des Auftretens ernährungsbedingter metabolischer sowie infektiöser Komplikationen in dieser Patientengruppe aus. Dies gilt insbesondere dann, wenn mit der parenteralen Ernährungslösung 28 bis 34 Energieprozent aus Aminosäuren, 41 bis 44 Energieprozent aus Glukose und etwa 25 bis 27 Energieprozent aus Kohlenhydraten bereitgestellt werden.
Durch den hohen Gehalt an Aminosäuren wird die Wundheilung begünstigt und das Auftreten einer negativen Stickstoffbilanz vermieden. Gleichzeitig kann durch die erfindungsgemäße parenterale Ernährungslösung eine Überversorgung des adipösen Intensivpatienten mit Lipiden und Kohlenhydraten wirksam verhindert werden, wie sie bei der Verabreichung von parenteralen Ernährungslösungen aus dem Stand der Technik, die auf die Bedürfnisse unter- oder normalgewichtiger Patienten abgestellt sind, unter Verwendung normaler Dosierungsschemata oder Anleitungen auftritt.
Bei Verabreichung der parenteralen Ernährungslösung werden Störungen des Glukosestoffwechsels wie Hyperglykämie oder Glukoseintoleranz vermieden oder vermindert, ebenso Störungen des Lipidstoffwechsels, wie das Auftreten einer Hypertriglyceridämie.
Dabei kommt es durch die besondere Zusammensetzung der parenteralen Ernährungslösung zu einem Zusammenwirken der für den adipösen Intensivpatienten vorteilhaften Effekte des hohen Aminosäuregehalts, sowie des vergleichsweise niedrigen Kohlenhydrat- und Lipidgehalt der parenteralen Ernährungslösung, der über einen bloßen additiven Effekt der drei Einzeleffekte hinausgeht. Dies gilt vor allem in Bezug auf die Verminderung der bei den adipösen Intensivpatienten beobachteten ernährungsbedingten Komplikationen wie Hypertriglyceridämie, Hyperglycämie und Störung des Aminosäurehaushalts, die durch die Verabreichung der parenteralen Sondennahrung wirksam vermieden oder vermindert werden können.

Als ganz besonders wirksam haben sich dabei solche parenteralen Ernährungslösungen herausgestellt die eine Aminosäurelösung, eine Glucoselösung und eine Lipidemulsion umfassen, und die pro mL 0,065 g bis 0,070 g Aminosäuren, 0,082 g bis 0,093 g Kohlenhydrate in Form von Glukose und 0,021 g bis 0,023 g Lipide umfassen, wobei die umfasste Aminosäurelösung pro 1000 mL der Aminosäurelösung etwa 5 g Isoleucin, etwa 7,4 g Leucin, etwa 9,31 g Lysinacetat (entsprechend etwa 6,6 g Lysin), etwa 4,3 g Methionin, etwa 5,1 g Phenylalanin, etwa 4,4 g Threonin, etwa 2 g Tryptophan, etwa 6,2 g Valin, etwa 12 g Arginin, etwa 3 g Histidin, etwa 14 g Alanin, etwa 11 g Glycin, etwa 11,2 g Prolin, etwa 6,5 g Serin, etwa 0,4 g Tyrosin und etwa 1 g Taurin umfasst, und wobei die umfasste Lipidemulsion pro 1000 mL etwa 60 g Sojaöl, etwa 60 g MCT, etwa 50 g Olivenöl und etwa 30 g Fischöl umfasst.

## Verabreichung/Dosierungsschemata

Die parenterale Ernährungslösung eignet sich hervorragend zur parenteralen Ernährung adipöser Intensivpatienten.

Dabei kann die parenterale Ernährungslösung als alleinige Nahrung, oder ergänzend zu einer enteralen oder oralen Ernährung des adipösen Intensivpatienten verabreicht werden.

Daneben können dem adipösen Intensivpatienten Elektrolyte, Spurenelemente und/oder Vitamine getrennt von der parenteralen Ernährungslösung zugeführt werden. Ganz besonders bevorzugt ist es aber, dass bei der Verabreichung der parenteralen Ernährungslösung Elektrolyte, Spurenelemente und/oder Vitamine in der parenteralen Ernährungslösung enthalten sind, so dass sie dem adipösen Intensivpatienten nicht getrennt von der parenteralen Ernährungslösung verabreicht werden müssen.

Die parenterale Ernährungslösung kann solange verabreicht werden, wie eine Indikation zur alleinigen oder ergänzenden parenteralen Ernährung besteht und der Intensivpatient adipös ist.

Beispielsweise kann die parenterale Ernährungslösung über einen Zeitraum von 1 Tag bis mehrere Monate an einen adipösen Intensivpatienten mit Indikation zur parenteralen Ernährung verabreicht werden. Die parenterale Ernährungslösung kann aufgrund ihrer besonderen, auf die ernährungsphysiologischen Bedürfnisse adipöser Intensivpatienten abgestimmten Zusammensetzung zur längerfristigen parenteralen Ernährung dieser Patienten eingesetzt werden, sogar dann, wenn die parenterale Ernährungslösung als alleinige Nahrung verabreicht wird. Die parenterale Ernährungslösung ist somit auch für eine Verabreichung über einen Zeitraum von 1 Monat bis 12 Monate, bevorzugt auch für eine Verabreichung über einen Zeitraum von 3 Monaten bis 12 Monaten, oder sogar von 6 Monaten bis 12 Monaten geeignet.

Es ist zu erwarten, dass der adipöse Patient unter Verabreichung der beschriebenen parenteralen Ernährung durch Mobilisierung körpereigener Energiereserven an Körpergewicht verliert. Die hier beschriebene parenterale Ernährungslösung sollte durch konventionelle parenterale Ernährung ersetzt werden, sobald der BMI des Patienten unter 30 kg/m$^2$ sinkt.

Die Verabreichung kann kontinuierlich, d.h. für 24 Stunden pro Tag ohne Unterbrechung erfolgen. Die Verabreichung kann aber auch diskontinuierlich erfolgen. Dabei wird die parenterale Ernährungslösung pro Tag dem adipösen Intensivpatienten beispielsweise für 16 Stunden verabreicht. Für die folgenden 8 Stunden erfolgt keine Verabreichung. Dann erfolgt erneut eine Verabreichung für 16 Stunden.

Die parenterale Ernährungslösung umfasst sowohl eine Aminosäurelösung als auch eine Glukoselösung als auch eine Lipidemulsion. Diese werden in einem Beutel bereitgestellt, daher wird eine einfache Dosierung ermöglicht. Mit der parenteralen Ernährungslösung können alle benötigten Makronährstoffe (Aminosäuren, Kohlenhydrate und Lipide) sowie, wenn gewünscht, auch alle benötigten Elektrolyte zugeführt werden. Spurenelemente und Vitamine können der parenteralen Ernährungslösung vor der Verabreichung zugesetzt werden.

Diese Verabreichungsform ist einfach und zeitsparend, sowie weniger anfällig für Fehlberechnungen oder Fehler bei der eigentlichen Verabreichung.

Anders als bei der parenteralen Ernährung mit getrennt zu verabreichenden Einzellösungen (getrennte Verabreichung einer Aminosäurelösung, einer Kohlenhydratlösung sowie einer Lipidemulsion) fallen keine Restvolumina der einzelnen Ernährungslösungen an, die später verworfen werden müssen. Dies gilt auch, wenn die parenterale Ernährungslösung über einen Dreikammerbeutel bereitgestellt wird, da hier zwar innerhalb des Dreikammerbeutels die in der parenteralen Ernährungslösung umfasste Aminosäurelösung, Glukoselösung und Lipidemulsion vor der Verabreichung in getrennten Kammern gelagert werden, die im Dreikammerbeutel umfassten Volumina der Aminosäurelösung, Glukoselösung und Lipidemulsion aber so aufeinander abgestimmt sind, dass keine Restvolumina von Einzellösungen anfallen.

Ist die parenterale Ernährungslösung in einem Dreikammerbeutel enthalten, der eine erste Kammer, eine zweite Kammer und eine dritte Kammer umfasst, wobei in der ersten Kammer die Aminosäurelösung umfasst ist, in der zweiten Kammer die Glukoselösung umfasst ist und in der dritten Kammer die Lipidemulsion umfasst ist, so sind die Aminosäurelösung, die Glukoselösung und die Lipidemulsion vor der Verabreichung der parenteralen Ernährungslösung an den adipösen Intensivpatienten zunächst miteinander zu vereinigen und zu mischen. Verfahren zur Mischung von in einem Dreikammerbeutel enthaltenen Lösungen zur parenteralen Ernährung sind dem Fachmann bekannt. Erst nach der Vereinigung und Durchmischung erfolgt die Verabreichung der parenteralen Ernährungslösung an den adipösen Intensivpatienten.

Der adipöse Intensivpatient kann ein Patient mit einer hyperkatabolen und/oder hypermetabolen Stoffwechsellage sein. Der adipöse Intensivpatient kann ein Patient sein, der kritisch krank ist und/oder ein Patient sein, der eine Erkrankung hat, die ausgewählt ist aus der Gruppe bestehend aus Trauma, Verbrennung, Infektion, Sepsis und chirurgischer Eingriff oder einer Kombination davon.

Der adipöse Intensivpatient kann ein Patient mit Adipositas Grad I, Adipositas Grad II oder Adipositas Grad III sein, und kann männlich oder weiblich sein.

Die täglich an den adipösen Intensivpatienten zu verabreichende Menge der parenteralen Ernährungslösung kann durch einen Parameter bestimmt werden, der ausgewählt ist aus der Gruppe bestehend aus Körpergröße des Patienten, Geschlecht des Patienten, Grad der Adipositas oder einer Kombination davon.

Der Grad der Adipositas kann basierend auf der Körpergröße und dem Körpergewicht des Patienten ermittelt werden. Alternativ kann der Grad der Adipositas auch basierend auf der Körperfettmenge und/oder der Körperfettverteilung des Patienten durch den Fachmann ermittelt werden.

Die Verabreichung der parenteralen Ernährungslösung nach folgendem Schema hat sich als besonders geeignet zur parenteralen Ernährung adipöser Intensivpatienten herausgestellt: Die parenterale Ernährungslösung wird, wenn der Patient an Adipositas Grad I oder Grad II leidet, dem Patienten in einer Menge von 28 mL bis 32 mL pro kg IBW und Tag verabreicht, besonders bevorzugt in einer Menge von 30 mL pro kg IBW und Tag.

Wenn der Patient an Adipositas Grad III leidet, wird die parenterale Ernährungslösung dem Patienten in einer Menge von 35 mL bis 39 mL pro kg IBW und Tag verabreicht, besonders bevorzugt in einer Menge von 37 mL pro kg IBW und Tag.

Ist nicht klar zu bestimmen, ob ein Patient unter Adipositas Grad II oder III leidet, sowie auch generell in der Übergangszone von Grad II zu Grad III, kann eine Dosierung zwischen 30 und 37 mL/kg gewählt werden.

Durch dieses Verabreichungsschema kann eine adäquate Nährstoffversorgung der Patienten sichergestellt werden. Gleichzeitig kann eine Überversorgung mit Makronährstoffen vermieden werden.

In einer ganz besonders bevorzugten Ausführungsform kann die täglich an den adipösen Intensivpatienten zu verabreichende Menge der parenteralen Ernährungslösung durch die Körpergröße, das Geschlecht und den Grad der Adipositas des adipösen Intensivpatienten bestimmt werden.

Ganz besonders bevorzugt ist es, wenn der Patient weiblich ist und an Adipositas Grad I oder Grad II leidet, dem Patienten pro Tag etwa 2100 mL der parenteralen Ernährungslösung bei einer Körpergröße von 180 cm, etwa 1950 mL bei einer Körpergröße von 174 cm, etwa 1800 mL bei einer Körpergröße von 168 cm, etwa 1650 mL bei einer Körpergröße von 163 cm oder etwa 1500 mL bei einer Körpergröße von 157 cm zu verabreichen. Wenn der Patient weiblich ist und an Adipositas Grad III leidet, ist es ganz besonders bevorzugt, dem Patienten pro Tag etwa 2590 mL der parenteralen Ernährungslösung bei einer Körpergröße von 180 cm, etwa 2405 mL bei einer Körpergröße von 174 cm, etwa 2220 mL bei einer Körpergröße von 168 cm, etwa 2035 mL bei einer Körpergröße von 163 cm oder etwa 1850 mL bei einer Körpergröße von 157 cm zu verabreichen.

Wenn der Patient männlich ist und an Adipositas Grad I oder Grad II leidet, ist es ganz besonders bevorzugt, dem Patienten pro Tag etwa 2550 mL der parenteralen Ernährungslösung bei einer Körpergröße von 187 cm, etwa 2400 mL bei einer Körpergröße von 182 cm, etwa 2250 mL bei einer Körpergröße von 178 cm, etwa 2100 mL bei einer Körpergröße von 173 cm, etwa 1950 mL bei einer Körpergröße von 168 cm oder etwa 1800 mL bei einer Körpergröße von 163 cm zu verabreichen. Wenn der Patient männlich ist und an Adipositas Grad III leidet, ist es ganz besonders bevorzugt, dem Patienten pro Tag etwa 3145 mL der parenteralen Ernährungslösung bei einer Körpergröße von 187 cm, etwa 2960 mL bei einer Körpergröße von 182 cm, etwa 2775 mL bei einer Körpergröße von 178 cm, etwa 2590 mL bei einer Körpergröße von 173 cm, etwa 2405 mL bei einer Körpergröße von 168 cm oder etwa 2220 mL bei einer Körpergröße von 163 cm zu verabreichen.

Unter Verwendung dieses ganz besonders bevorzugten Verabreichungsschemas kann das Auftreten von ernährungsbedingten metabolischen und infektiösen Komplikationen besonders wirksam vermieden werden, und die Morbiditäts- sowie Mortalitätsrate der adipösen Intensivpatienten besonders wirksam gesenkt werden.

Die vorstehend genannten Verabreichungsschemata beziehen sich auf eine Verabreichung der parenteralen Ernährungslösung als alleinige parenterale Nahrung. Wird der adipöse Intensivpatient zusätzlich enteral und/oder oral ernährt, so wird der prozentuale Anteil der durch die parenterale Nahrung zugeführten Energiemenge an der durch die Nahrung insgesamt pro Tag zugeführten Energiemenge berechnet, und nur der entsprechende prozentuale Anteil gemäß den vorstehend genannten Verabreichungsschemata verabreicht.

**Beispiele**

**Beispiel 1: Herstellung einer parenteralen Ernährungslösung**

**a) Die folgenden parenteralen Ernährungslösungen werden hergestellt:**

| Lösung | A0 | B0 | C0 | D0 | E0 | F0 |
|---|---|---|---|---|---|---|
| Aminosäuren [g/L] | 50 | 60 | 83 | 67 | 65 | 63 |
| Glukose [g/L] | 67 | 67 | 111 | 89 | 89 | 89 |
| Lipide [g/L] | 17 | 17 | 28 | 22 | 22 | 22 |

**b) Die folgenden parenteralen Ernährungslösungen werden hergestellt:**

| Lösung | A1 | B1 | C1 | D1 | E1 | F1 |
|---|---|---|---|---|---|---|
| Aminosäuren [g/L] | 50 | 60 | 83 | 67 | 65 | 63 |
| Glukose [g/L] | 67 | 67 | 111 | 89 | 89 | 89 |
| Lipide [g/L] | 17 | 17 | 28 | 22 | 22 | 22 |
| Elektrolyte[1] | + | + | + | + | + | + |

[1] Die Lösungen A1 bis F1 umfassen im Gegensatz zu den Lösungen A0 bis F0 aus Beispiel 1 a) zugesetzte Elektrolyte. In den Lösungen A1 bis F1 sind pro L 41 mmol Natrium, 30 mmol Kalium, 5,1 mmol Magnesium, 2,5 mmol Calcium, 10,2 mmol Phosphat, 0,04 mmol Zink, 5,1 mmol Sulfat, 36 mmol Chlorid und 106 mmol Acetat zugesetzt.

**c) Herstellung der Lösungen A0 bis F1 unter Verwendung einer besonders bevorzugten Aminosäurezusammensetzung**

Die Lösungen A0 bis F1 werden wie unter a) und b) beschrieben hergestellt. Dabei wird als Aminosäurequelle eine Aminosäurelösung verwendet, die pro 1000 mL 5 g Isoleucin, 7,4 g Leucin, 9,31 g Lysinacetat (entsprechend 6,6 g Lysin), 4,3 g Methionin, 5,1 g Phenylalanin, 4,4 g Threonin, 2 g Tryptophan, 6,2 g Valin, 12 g Arginin, 3 g Histidin, 14 g Alanin, 11 g Glycin, 11,2 g Prolin, 6,5 g Serin, 0,4 g Tyrosin und 1 g Taurin enthält.
Die so erhaltenen Lösungen werden mit dem Zusatz "AS" versehen und als A0AS bis F1AS bezeichnet.

**d) Herstellung der Lösungen A0 bis F1 unter Verwendung einer besonders bevorzugten Lipidzusammensetzung**

Die Lösungen A0 bis F1 werden wie unter a) und b) beschrieben hergestellt. Dabei wird als Lipidquelle eine Lipidemulsion verwendet, die pro 1000 mL 60 g Sojaöl, 60 g MCT, 50 g Olivenöl und 30 g Fischöl enthält.
Die so erhaltenen Lösungen werden mit dem Zusatz "LP" versehen und als A0LP bis F1LP bezeichnet.

**e) Herstellung der Lösungen A0 bis F1 unter Verwendung der besonders bevorzugten Aminosäure- und Lipidzusammensetzung**

Die Lösungen A0 bis F1 werden wie unter a) und b) beschrieben hergestellt. Dabei wird als Aminosäurequelle eine Aminosäurelösung verwendet, die pro 1000 mL 5 g Isoleucin, 7,4 g Leucin, 9,31 g Lysinacetat (entsprechend 6,6 g Lysin), 4,3 g Methionin, 5,1 g Phenylalanin, 4,4 g Threonin, 2 g Tryptophan, 6,2 g Valin, 12 g Arginin, 3 g Histidin, 14 g Alanin, 11 g Glycin, 11,2 g Prolin, 6,5 g Serin, 0,4 g Tyrosin und 1 g Taurin enthält.
Als Lipidquelle wird eine Lipidemulsion verwendet, die pro 1000 mL 60 g Sojaöl, 60 g MCT, 50 g Olivenöl und 30 g Fischöl enthält.
Die so erhaltenen Lösungen werden mit dem Zusatz "AS-LP" versehen und als A0AS-LP bis F1AS-LP bezeichnet.

**Beispiel 2: Dreikammerbeutel**

Die folgenden Dreikammerbeutel werden hergestellt:

| Dreikammerbeutel | 1 | 2 | 3 |
|---|---|---|---|
| Gesamtvolumen der parenteralen Ernährungslösung [mL] | 2250 | 1125 | 3375 |
| Volumen der Lösung in Kammer 1 [mL] | 1500 | 750 | 2250 |
| Volumen der Lösung in Kammer 2 [mL] | 500 | 250 | 750 |
| Volumen der Lösung in Kammer 3 [mL] | 250 | 125 | 375 |

Kammer 1 des Dreikammerbeutels enthält eine Aminosäurenlösung (100 g Aminosäuren/L), Kammer 2 eine Glukoselösung (400 g Glukose/L) und Kammer 3 eine Lipidemulsion (200 g Lipide/L). Bezogen auf das Gesamtvolumen der parenteralen Ernährungslösung in dem Dreikammerbeuteln 1, 2 und 3 liegen 67 g/L Aminosäuren, 89 g/L Glukose und 22 g/L Lipide vor. Kammern 1 und/oder 2 können zusätzlich Elektrolyte enthalten.

Die Aminosäureemulsion kann pro 1000 mL die folgende Zusammensetzung aufweisen: 5 g Isoleucin, 7,4 g Leucin, 9,31 g Lysinacetat (entsprechend 6,6 g Lysin), 4,3 g Methionin, 5,1 g Phenylalanin, 4,4 g Threonin, 2 g Tryptophan, 6,2 g Valin, 12 g Arginin, 3 g Histidin, 14 g Alanin, 11 g Glycin, 11,2 g Prolin, 6,5 g Serin, 0,4 g Tyrosin und 1 g Taurin.

Die Lipidemulsion kann pro 1000 mL die folgende Zusammensetzung aufweisen: 60 g Sojaöl, 60 g MCT, 50 g Olivenöl und 30 g Fischöl.

**Beispiel 3: Dosierungsschemata**

Die folgende Tabelle stellt ein beispielhaftes Dosierungsschema dar, nach dem die erfindungsgemäße parenterale Ernährungslösung an adipöse Intensivpatienten verabreicht werden kann. Das Idealkörpergewicht von männlichen und weiblichen Patienten kann, basierend auf der Körpergröße der adipösen Intensivpatienten, der Tabelle entnommen werden, ebenso ist die empfohlene Dosierung der parenteralen Ernährungslösung basierend auf der Körpergröße des Patienten der Tabelle direkt zu entnehmen.

| Dosierungsschema | | | | |
|---|---|---|---|---|
| Körpergröße (cm) | | | Erfindungsgemäße parenterale Ernährungslösung (Volumen/Tag in ml) | |
| Männer | Frauen | IBW* | Adipositas Grad I, | Adipositas Grad |
| 187 | | 85 | 2550 ml | 3145 ml |
| 182 | | 80 | 2400 | 2960 |
| 178 | | 75 | 2250 | 2775 |
| 173 | 180 | 70 | 2100 | 2590 |
| 168 | 174 | 65 | 1950 | 2405 |
| 163 | 168 | 60 | 1800 | 2220 |
| | 163 | 55 | 1650 | 2035 |
| | 157 | 50 | 1500 | 1850 |
| * IBW = ideal body weight; Idealgewicht | | | | |

**Beispiel 4: Verabreichungsbeispiele**

**a) Verabreichung der parenteralen Ernährungslösung an einen adipösen männlichen Intensivpatienten mit 178 cm Körpergröße.**

Mit Hilfe des tatsächlichen Körpergewichts von 150 kg lässt sich für den 178 cm großen Patienten ein BMI von 47,3 berechnen. Der Patient hat folglich Adipositas Grad III. Alternativ kann der Grad der Adipositas auch basierend auf Körperfettanteil und/oder Körperfettverteilung des Patienten ermittelt werden., was für das mit der Verabreichung von parenteralen Ernährungslösungen betraute Klinikpersonal kein Problem darstellt. Aus der in Beispiel 3 dargestellten Tabelle ergibt sich somit für diesen Patienten ein tägliches Infusionsvolumen von 2775 mL der parenteralen Ernährungslösung.

**b) Verabreichung der parenteralen Ernährungslösung an eine adipösen weiblichen Intensivpatientin mit 163 cm Körpergröße.**

Mit Hilfe des tatsächlichen Körpergewichts von 100 kg lässt sich für die 163 cm große Patientin ein BMI von 37,6 berechnen. Die Patientin hat folglich Adipositas Grad II. Alternativ kann der Grad der Adipositas auch basierend auf Körperfettanteil und/oder Körperfettverteilung des Patienten ermittelt werden. Aus der in Beispiel 3 dargestellten Tabelle ergibt sich somit für diese Patientin ein tägliches Infusionsvolumen von 1800 mL der parenteralen Ernährungslösung.

### Beispiel 5: Einfluss auf Komplikationen, Morbidität und Mortalität

Die Lösungen A0-F0 oder A1-F1 werden gemäß dem in Beispiel 3 dargestellten Dosierungsschema an männliche und weibliche adipöse Intensivpatienten mit Adipositas Grad I, Grad II oder Grad III für einen Zeitraum von 2 Wochen oder 2 Monaten als alleinige Nahrung parenteral verabreicht. Wird eine der Lösungen A0 - F0 verabreicht, werden der parenteralen Ernährungslösung vor der Verabreichung an den Patienten zusätzlich Elektrolyte, Spurenelemente und Vitamine in bedarfsdeckender Menge zugesetzt. Wird eine der Lösungen A1 - F1 verabreicht, werden der parenteralen Ernährungslösung vor der Verabreichung an den Patienten zusätzlich Spurenelemente und Vitamine in bedarfsdeckender Menge zugesetzt.

Während des Verabreichungszeitraums ist das Auftreten von ernährungsbedingten Komplikationen wie Hyperglycämie oder Hypertriglyceridämie unwahrscheinlich. Eine ausgeglichene Stickstoffbilanz kann durch die Ernährung erreicht werden.

Beobachtet wird eine leichte Gewichtsreduktion der Patienten, unter vorwiegendem Erhalt der fettfreien Körpermasse.

Es kommt zu einem verminderten Auftreten von metabolischen Komplikationen. Damit einhergehend kann die durchschnittliche Verweildauer auf der Intensivstation gegenüber parenteral ernährten adipösen Intensivpatienten, die eine auf die Bedürfnisse untergewichtiger kataboler Patienten abgestimmte Vergleichlösung erhalten, verkürzt sein.

Alle Lösungen sind gut zur Verabreichung an adipöse Intensivpatienten geeignet. Dabei waren Lösungen mit höherem Aminosäuregehalt (die Lösungen B0-F0 und B1-F1) besonders wirksam. Am besten schneiden die Lösungen mit einem Aminosäuregehalt von 67 g/L ab, d. h. die Lösungen D0 und D1.

Besonders gute Erfolge hinsichtlich einer Reduktion der metabolischen Komplikationen werden erzielt, wenn Lösungen des Typs AS-LP verabreicht werden, d.h. A0 AS-LP - F0 AS-LP oder A1 AS-LP - F1 AS-LP. Hier wird ein besonders ausgeprägter mehr als additiver günstiger Effekt in Form einer verminderten Häufigkeit des Auftretens von metabolischen und infektiösen Komplikationen im Vergleich zu Lösungen des Typs AS (A0 AS - F0 AS oder A1 AS - F1 AS) oder LP (A0 LP - F0 LP oder A1 LP - F1 LP) alleine beobachtet.

### Beispiel 6: Vergleich

Die Lösungen A1 - F1 und A1 AS-LP - F1 AS-LP werden gemäß dem in Beispiel 3 dargestellten Dosierungsschema an männliche und weibliche adipöse Intensivpatienten mit Adipositas Grad I, Grad II oder Grad III für einen Zeitraum von 2 Wochen oder 2 Monaten als alleinige Nahrung parenteral verabreicht. Den parenteralen Ernährungslösungen werden vor der Verabreichung an den Patienten zusätzlich Spurenelemente und Vitamine in bedarfsdeckender Menge zugesetzt.

Das Auftreten und/oder der Schweregrad auftretender metabolischer und infektiöser Komplikationen ist bei adipösen Intensivpatienten, die eine der Lösungen A1 - F1 und A1 AS-LP - F1 AS-LP erhalten, geringer als bei Patienten, die eine Vergleichslösung mit identischem Aminosäuregehalt bei gleichzeitig hohem Lipidgehalt von 40 g/L erhalten. Der Effekt ist besonders ausgeprägt, wenn eine der Lösungen A1 AS-LP - F1 AS-LP verabreicht wird, insbesondere bei Lösung D1 AS-LP.

### Patentansprüche

1. Parenterale Ernährungslösung zur Verwendung in der Verabreichung an einen adipösen Intensivpatienten, die Ernährungslösung umfassend pro 1000 ml:

   (i) 500 ml - 834 ml einer Aminosäurelösung umfassend 50 g - 83 g Aminosäuren,
   (ii) 167 ml - 278 ml einer Glukoselösung umfassend 67 g - 111 g Glukose, und

(iii) 83 ml - 139 ml einer Lipidemulsion umfassend 17 g - 28 g Lipide, wobei, wenn der Patient an Adipositas Grad I oder Grad II leidet, dem Patienten pro Tag 28 ml bis 32 ml pro kg IBW, besonders bevorzugt 30 ml pro kg IBW verabreicht werden, oder wobei, wenn der Patient an Adipositas Grad III leidet, dem Patienten pro Tag 35 ml bis 39 ml pro kg IBW, besonders bevorzugt 37 ml pro kg IBW verabreicht werden.

2.  Parenterale Ernährungslösung zur Verwendung gemäß Anspruch 1, die Ernährungslösung umfassend pro 1000 ml:

    (i) 667 ml einer Aminosäurelösung umfassend 67 g Aminosäuren,
    (ii) 222 ml einer Glukoselösung umfassend 89 g Glukose,
    und
    (iii) 111 ml einer Lipidemulsion umfassend 22 g Lipide.

3.  Parenterale Ernährungslösung zur Verwendung gemäß einem der vorstehenden Ansprüche, wobei die Aminosäurelösung pro 1000 ml der Aminosäurelösung mindestens einen Bestandteil umfasst, der ausgewählt ist aus der Gruppe bestehend aus:

    etwa 5 g Isoleucin, etwa 7,4 g Leucin, etwa 9,31 g Lysinacetat (entsprechend etwa 6,6 g Lysin), etwa 4,3 g Methionin, etwa 5,1 g Phenylalanin, etwa 4,4 g Threonin, etwa 2 g Tryptophan, etwa 6,2 g Valin, etwa 12 g Arginin, etwa 3 g Histidin, etwa 14 g Alanin, etwa 11 g Glycin, etwa 11,2 g Prolin, etwa 6,5 g Serin, etwa 0,4 g Tyrosin 25 und etwa 1 g Taurin oder einer Kombination davon,
    und/oder
    wobei die Lipidemulsion pro 1000 ml der Lipidemulsion mindestens einen Bestandteil umfasst, der ausgewählt ist aus der Gruppe bestehend aus:
    etwa 60 g Sojaöl, etwa 60 g MCT, etwa 50 g Olivenöl und etwa 30 g Fischöl oder einer Kombination davon.

4.  Parenterale Ernährungslösung zur Verwendung gemäß einem der vorstehenden Ansprüche, wobei die parenterale Ernährungslösung ferner mindestens einen Bestandteil umfasst, der ausgewählt ist aus der Gruppe bestehend aus: Elektrolyte, Dipeptide, Spurenelemente, Vitamine oder einer Kombination davon.

5.  Parenterale Ernährungslösung zur Verwendung gemäß Anspruch 4, wobei die parenterale Ernährungslösung pro 1000 ml der Ernährungslösung Elektrolyte umfasst, die ausgewählt sind aus der Gruppe bestehend aus etwa 32,8 mmol Natrium bis etwa 48 mmol Natrium, etwa 24 mmol Kalium bis etwa 36 mmol Kalium, etwa 4,1 mmol Magnesium bis etwa 6,1 mmol Magnesium, etwa 2 mmol Calcium bis etwa 3 mmol Calcium, etwa 8,2 mmol Phosphat bis etwa 15,6 mmol Phosphat, etwa 0,032 mmol Zink bis etwa 0,048 mmol Zink, etwa 4,1 mmol Sulfat bis etwa 6,1 mmol Sulfat, etwa 28,8 mmol Chlorid bis etwa 43,2 mmol Chlorid, etwa 84,8 mmol Acetat bis etwa 127,2 mmol Acetat oder einer Kombination davon,
    bevorzugt wobei die parenterale Ernährungslösung pro 1000 ml der Ernährungslösung Elektrolyte umfasst, die ausgewählt sind aus der Gruppe bestehend aus etwa 41 mmol Natrium, etwa 30 mmol Kalium, etwa 5,1 mmol Magnesium, etwa 2,5 mmol Calcium, etwa 13 mmol Phosphat, etwa 0,04 mmol Zink, etwa 5,1 mmol Sulfat, etwa 36 mmol Chlorid, etwa 106 mmol Acetat oder einer Kombination davon.

6.  Parenterale Ernährungslösung zur Verwendung gemäß einem der vorstehenden Ansprüche, wobei die Ernährungslösung pro 100 Energieprozent 25 - 35 Energieprozent aus Aminosäuren, 40 - 45 Energieprozent aus Glucose und 22 - 30 Energieprozent aus Lipiden umfasst,

    bevorzugt wobei die Ernährungslösung pro 100 Energieprozent 28 - 34 Energieprozent aus Aminosäuren, 41 - 44 Energieprozent aus Glucose und 25 - 27 Energieprozent aus Lipiden umfasst,
    am meisten bevorzugt wobei die Ernährungslösung pro 100 Energieprozent 32 Energieprozent aus Aminosäuren, 42 Energieprozent aus Glucose und 26 Energieprozent aus Lipiden umfasst.

7.  Parenterale Ernährungslösung zur Verwendung gemäß einem der vorstehenden Ansprüche, wobei die Ernährungslösung pro 1000 ml einen Energiegehalt von 700 kcal - 975 kcal hat,
    bevorzugt wobei die Ernährungslösung pro 1000 ml einen Energiegehalt von 800 kcal bis 880 kcal hat, am meisten bevorzugt, wobei die Ernährungslösung pro 1000 ml einen Energiegehalt von 820 kcal - 860 kcal hat.

8.  Parenterale Ernährungslösung zur Verwendung gemäß einem der vorstehenden Ansprüche, wobei die parenterale Ernährungslösung in einem Dreikammerbeutel umfassend eine erste Kammer, eine zweite Kammer und eine dritte Kammer enthalten ist, der

(i) in der ersten Kammer die Aminosäurelösung umfasst,
(ii) in der zweiten Kammer die Glukoselösung umfasst,
und
(iii) in der dritten Kammer die Lipidemulsion umfasst,

wobei die Aminosäurelösung, die Glukoselösung und die Lipidemulsion vor der Verwendung vereinigt und gemischt werden.

9. Parenterale Ernährungslösung zur Verwendung gemäß einem der vorstehenden Ansprüche, wobei der htensivpatient ein Patient mit einer hyperkatabolen und/oder hypermetabolen Stoffwechsellage ist,
bevorzugt wobei der Intensivpatient ein Patient ist, der kritisch krank ist und/oder ein Patient ist, der eine Erkrankung hat, die ausgewählt ist aus der Gruppe bestehend aus: Trauma, Verbrennung, Infektion, Sepsis und chirurgischer Eingriff oder einer Kombination davon.

10. Parenterale Ernährungslösung zur Verwendung gemäß einem der vorstehenden Ansprüche, wobei die Adipositas ausgewählt ist aus der Gruppe bestehend aus: Adipositas Grad I, Adipositas Grad II und Adipositas Grad III.

11. Parenterale Ernährungslösung zur Verwendung gemäß einem der vorstehenden Ansprüche, wobei die täglich zu verabreichende Menge der parenteralen Ernährungslösung durch einen Parameter bestimmt wird, der ausgewählt ist aus der Gruppe bestehend aus:
Körpergröße des Patienten, Geschlecht des Patienten, Grad der Adipositas oder einer Kombination davon.

12. Parenterale Ernährungslösung zur Verwendung gemäß einem der vorstehenden Ansprüche,

wobei, wenn der Patient weiblich ist und an Adipositas Grad I oder Grad II leidet, dem Patienten pro Tag etwa 2100 ml der parenteralen Ernährungslösung bei einer Körpergröße von 180 cm, etwa 1950 ml bei einer Körpergröße von 174 cm, etwa 1800 ml bei einer Körpergröße von 168 cm, etwa 1650 ml bei einer Körpergröße von 163 cm oder etwa 1500 ml bei einer Körpergröße von 157 cm zu verabreichen sind,
oder
wobei, wenn der Patient weiblich ist und an Adipositas Grad III leidet, dem Patienten pro Tag etwa 2590 ml der parenteralen Ernährungslösung bei einer Körpergröße von 180 cm, etwa 2405 ml bei einer Körpergröße von 174 cm, etwa 2220 ml bei einer Körpergröße von 168 cm, etwa 2035 ml bei einer Körpergröße von 163 cm oder etwa 1850 ml bei einer Körpergröße von 157 cm zu verabreichen sind,
oder
wobei, wenn der Patient männlich ist und an Adipositas Grad I oder Grad II leidet, dem Patienten pro Tag etwa 2550 ml der parenteralen Ernährungslösung bei einer Körpergröße von 187 cm, etwa 2400 ml bei einer Körpergröße von 182 cm, etwa 2250 ml bei einer Körpergröße von 178 cm, etwa 2100 ml bei einer Körpergröße von 173 cm, etwa 1950 ml bei einer Körpergröße von 168 cm oder etwa 1800 ml bei einer Körpergröße von 163 cm zu verabreicht sind,
oder
wobei, wenn der Patient männlich ist und an Adipositas Grad III leidet, dem Patienten pro Tag etwa 3145 ml der parenteralen Ernährungslösung bei einer Körpergröße von 187 cm, etwa 2960 ml bei einer Körpergröße von 182 cm, etwa 2775 ml bei einer Körpergröße von 178 cm, etwa 2590 ml bei einer Körpergröße von 173 cm, etwa 2405 ml bei einer Körpergröße von 168 cm oder etwa 2220 ml bei einer Körpergröße von 163 cm zu verabreichen sind.

13. Dreikammerbeutel umfassend eine erste Kammer, eine zweite Kammer und eine dritte Kammer,

die erste Kammer umfassend 750 ml - 2250 ml einer Aminosäurelösung umfassend 56,3 g - 281,3 g Aminosäuren,
die zweite Kammer umfassend 250 ml - 750 ml einer Glukoselösung umfassend 75 g - 375 g Glukose, und
die dritte Kammer umfassend 125 ml - 375 ml einer Lipidemulsion umfassend 18,8 g - 93,8 g Lipide,
wobei der Dreikammerbeutel, bezogen auf das Gesamtvolumen der ersten, der zweiten und der dritten Kammer, pro 1000 ml mindestens 50 g Aminosäuren umfasst, bevorzugt wobei der Dreikammerbeutel, bezogen auf das Gesamtvolumen der ersten, der zweiten und der dritten Kammer, pro 1000 ml mindestens 60 g Aminosäuren, besonders bevorzugt mindestens 63 g Aminosäuren umfasst, und wobei die Lipidemulsion pro 1000 ml mindestens einen Bestandteil umfasst, der ausgewählt ist aus der Gruppe bestehend aus etwa 60 g Sojaöl, etwa 60 g MCT, etwa 50 g Olivenöl und etwa 30 g Fischöl oder einer Kombination davon.

**14.** Dreikammerbeutel gemäß Anspruch 13,

wobei die erste Kammer 1500 ml einer Aminosäurelösung umfassend 135 g - 187,5 g Aminosäuren, bevorzugt 150 g Aminosäuren, umfasst,

die zweite Kammer 500 ml einer Glukoselosung umfassend 150 g - 250 g Glucose, bevorzugt 200 g Glucose, umfasst,
und die dritte Kammer 250 ml einer Lipidemulsion umfassend 37,5 g - 62,5 g Lipide, bevorzugt 50 g Lipide, umfasst,
oder

wobei die erste Kammer 750 ml einer Aminosäurelösung umfassend 67,5 g - 93,8 g Aminosäuren, bevorzugt 75 g Aminosäuren, umfasst,

die zweite Kammer 250 ml einer Glukoselösung umfassend 75 g - 125 g Glucose, bevorzugt 100 g Glucose, umfasst,
und die dritte Kammer 125 ml einer Lipidemulsion umfassend 18,8 g - 31,3 g Lipide, bevorzugt 25 g Lipide, umfasst,
oder

wobei die erste Kammer 2250 ml einer Aminosäurelösung umfassend 202,5 g - 281 g Aminosäuren, bevorzugt 225 g Aminosäuren, umfasst,

die zweite Kammer 750 ml einer Glukoselösung umfassend 225 g - 375 g Glucose, bevorzugt 300 g Glucose umfasst,
und die dritte Kammer 375 ml einer Lipidemulsion umfassend 56,3 g - 93,8 g Lipide, bevorzugt 75 g Lipide, umfasst.

**Claims**

**1.** A parenteral nutrition solution for use in administration to an obese intensive-care patient, the nutrition solution comprising, per 1000 ml:

(i) 500 ml to 834 ml of an amino acid solution comprising 50 g to 83 g of amino acids,
(ii) 167 ml to 278 ml of a glucose solution comprising 67 g to 111 g of glucose, and
(iii) 83 ml to 139 ml of a lipid emulsion comprising 17 g to 28 g of lipids,

wherein, if the patient suffers from class I or class II obesity, 28 ml to 32 ml per kg of IBW, particularly preferably 30 ml per kg of IBW are administered to the patient per day, or wherein, if the patient suffers from class III obesity, 35 ml to 39 ml per kg of IBW, particularly preferably 37 ml per kg of IBW are administered to the patient per day.

**2.** The parenteral nutrition solution for the use according to claim 1, the nutrition solution comprising, per 1000 ml:

(i) 667 ml of an amino acid solution comprising 67 g of amino acids,
(ii) 222 ml of a glucose solution comprising 89 g of glucose, and
(iii) 111 ml of a lipid emulsion comprising 22 g of lipids.

**3.** The parenteral nutrition solution for the use according to one of the preceding claims, wherein the amino acid solution comprises per 1000 ml of the amino acid solution, at least one constituent, selected from the group consisting of:

approximately 5 g of isoleucine, approximately 7.4 g of leucine, approximately 9.31 g of lysine acetate (corresponding to approximately 6.6 g of lysine), approximately 4.3 g of methionine, approximately 5.1 g of phenylalanine, approximately 4.4 g of threonine, approximately 2 g of tryptophan, approximately 6.2 g of valine, approximately 12 g of arginine, approximately 3 g of histidine, approximately 14 g of alanine, approximately 11 g of glycine, approximately 11.2 g of proline, approximately 6.5 g of serine, approximately 0.4 g of tyrosine 25 and approximately 1 g of taurine or a combination thereof,
and/or

wherein the lipid emulsion comprises, per 1000 ml of the lipid emulsion, at least one constituent, selected from the group consisting of:

approximately 60 g of soya oil, approximately 60 g of MCTs, approximately 50 g of olive oil and approximately 30 g of fish oil or a combination thereof.

4. The parenteral nutrition solution for the use according to one of the preceding claims, wherein the parenteral nutrition solution further comprises at least one constituent, selected from the group consisting of:
electrolytes, dipeptides, trace elements, vitamins or a combination thereof.

5. The parenteral nutrition solution for the use according to claim 4, wherein the parenteral nutrition solution comprises, per 1000 ml of the nutrition solution, electrolytes selected from the group consisting of approximately 32.8 mmol of sodium to approximately 48 mmol of sodium, approximately 24 mmol of potassium to approximately 36 mmol of potassium, approximately 4.1 mmol of magnesium to approximately 6.1 mmol of magnesium, approximately 2 mmol of calcium to approximately 3 mmol of calcium, approximately 8.2 mmol of phosphate to approximately 15.6 mmol of phosphate, approximately 0.032 mmol of zinc to approximately 0.048 mmol of zinc, approximately 4.1 mmol of sulphate to approximately 6.1 mmol of sulphate, approximately 28.8 mmol of chloride to approximately 43.2 mmol of chloride, approximately 84.8 mmol of acetate to approximately 127.2 mmol of acetate or a combination thereof, wherein preferably the parenteral nutrition solution comprises, per 1000 ml of the nutrition solution, electrolytes, selected from the group consisting of approximately 41 mmol of sodium, approximately 30 mmol of potassium, approximately 5.1 mmol of magnesium, approximately 2.5 mmol of calcium, approximately 13 mmol of phosphate, approximately 0.04 mmol of zinc, approximately 5.1 mmol of sulphate, approximately 36 mmol of chloride, approximately 106 mmol of acetate or a combination thereof.

6. The parenteral nutrition solution for the use according to one of the preceding claims, wherein the nutrition solution comprises, per 100% of energy, 25 to 35% of energy from amino acids, 40 to 45% of energy from glucose and 22 to 30% of energy from lipids,

wherein preferably the nutrition solution comprises, per 100% of energy, 28 to 34% of energy from amino acids, 41 to 44% of energy from glucose and 25 to 27% of energy from lipids,
wherein most preferably the nutrition solution comprises, per 100% of energy, 32% of energy from amino acids, 42% of energy from glucose and 26% of energy from lipids.

7. The parenteral nutrition solution for the use according to one of the preceding claims, wherein the nutrition solution has an energy content of 700 kcal to 975 kcal per 1000 ml,
wherein preferably the nutrition solution has an energy content of 800 kcal to 880 kcal per 1000 ml, wherein most preferably the nutrition solution has an energy content of 820 kcal to 860 kcal per 1000 ml.

8. The parenteral nutrition solution for the use according to one of the preceding claims, wherein the parenteral nutrition solution is contained in a three-chamber pouch comprising a first chamber, a second chamber and a third chamber, with (i) the first chamber comprising the amino acid solution, (ii) the second chamber comprising the glucose solution, and (iii) the third chamber comprising the lipid emulsion, wherein the amino acid solution, the glucose solution and the lipid emulsion are combined and mixed prior to use.

9. The parenteral nutrition solution for the use according to one of the preceding claims, wherein the intensive-care patient is a patient in a hypercatabolic state and/or hypermetabolic state,
wherein preferably the intensive-care patient is a patient, who is critically ill and/or is a patient, who has a condition, selected from the group consisting of: trauma, burn, infection, sepsis and surgical operation or a combination thereof.

10. The parenteral nutrition solution for the use according to one of the preceding claims, the obesity is selected from the group consisting of: class I obesity, class II obesity and class III obesity.

11. The parenteral nutrition solution for the use according to one of the preceding claims, wherein the amount of the parenteral nutrition solution to be administered daily is determined by a parameter, selected from the group consisting of:
height of the patient, gender of the patient, degree of obesity or a combination thereof.

12. The parenteral nutrition solution for the use according to one of the preceding claims,

wherein, if the patient is female and suffers from class I or class II obesity, approximately 2100 ml of the parenteral nutrition solution in the case of a height of 180 cm, approximately 1950 ml in the case of a height of 174 cm, approximately 1800 ml in the case of a height of 168 cm, approximately 1650 ml in the case of a height of 163 cm or approximately 1500 ml in the case of a height of 157 cm are to be administered to the patient per day, or

wherein, if the patient is female and suffers from class III obesity, approximately 2590 ml of the parenteral nutrition solution in the case of a height of 180 cm, approximately 2405 ml in the case of a height of 174 cm, approximately 2220 ml in the case of a height of 168 cm, approximately 2035 ml in the case of a height of 163 cm or approximately 1850 ml in the case of a height of 157 cm are to be administered to the patient per day, or

wherein, if the patient is male and suffers from class I or class II obesity, approximately 2550 ml of the parenteral nutrition solution in the case of a height of 187 cm, approximately 2400 ml in the case of a height of 182 cm, approximately 2250 ml in the case of a height of 178 cm, approximately 2100 ml in the case of a height of 173 cm, approximately 1950 ml in the case of a height of 168 cm or approximately 1800 ml in the case of a height of 163 cm are to be administered to the patient per day, or

wherein, if the patient is male and suffers from class III obesity, approximately 3145 ml of the parenteral nutrition solution in the case of a height of 187 cm, approximately 2960 ml in the case of a height of 182 cm, approximately 2775 ml in the case of a height of 178 cm, approximately 2590 ml in the case of a height of 173 cm, approximately 2405 ml in the case of a height of 168 cm or approximately 2220 ml in the case of a height of 163 cm are to be administered to the patient per day.

13. A three-chamber pouch comprising a first chamber, a second chamber and a third chamber,

the first chamber comprising 750 ml to 2250 ml of an amino acid solution comprising 56.3 g to 281.3 g of amino acids,
the second chamber comprising 250 ml to 750 ml of a glucose solution comprising 75 g to 375 g of glucose, and third chamber comprising 125 ml to 375 ml of a lipid emulsion comprising 18.8 g to 93.8 g of lipids,
wherein the three-chamber pouch, based on the total volume of the first, the second chamber and the third chamber, comprises at least 50 g of amino acids per 1000 ml,

wherein preferably the three-chamber pouch, based on the total volume of the first, the second chamber and the third chamber, comprises at least 60 g of amino acids per 1000 ml, particularly preferably at least 63 g of amino acids, and wherein the lipid emulsion comprises, per 1000 ml, at least one constituent, selected from the group consisting of approximately 60 g of soya oil, approximately 60 g of MCTs, approximately 50 g of olive oil and approximately 30 g of fish oil or a combination thereof.

14. The three-chamber pouch according to claim 13,

wherein the first chamber comprises 1500 ml of an amino acid solution comprising 135 g to 187.5 g of amino acids, preferably 150 g of amino acids,
the second chamber comprises 500 ml of a glucose solution comprising 150 g to 250 g of glucose, preferably 200 g of glucose,
and the third chamber comprises 250 ml of a lipid emulsion comprising 37.5 g to 62.5 g of lipids, preferably 50 g of lipids,
or

wherein the first chamber comprises 750 ml of an amino acid solution comprising 67.5 g to 93.8 g of amino acids, preferably 75 g of amino acids,
the second chamber comprises 250 ml of a glucose solution comprising 75 g to 125 g of glucose, preferably 100 g of glucose,
and the third chamber comprises 125 ml of a lipid emulsion comprising 18.8 g to 31.3 g of lipids, preferably 25 g of lipids,
or

wherein the first chamber comprises 2250 ml of an amino acid solution comprising 202.5 g to 281 g of amino acids, preferably 225 g of amino acids,

the second chamber comprises 750 ml of a glucose solution comprising 225 g to 375 g of glucose, preferably 300 g of glucose,
and the third chamber comprises 375 ml of a lipid emulsion comprising 56.3 g to 93.8 g of lipids, preferably 75 g of lipids.

**Revendications**

1. Solution de nutrition parentérale destinée à être utilisée dans l'administration à un patient obèse en soins intensifs, la solution de nutrition comprenant, pour 1000 ml :

   (i) 500 ml à 834 ml d'une solution d'acides aminés comprenant 50 g à 83 g d'acides aminés,
   (ii) 167 ml à 278 ml d'une solution de glucose comprenant 67 g à 111 g de glucose, et
   (iii) 83 ml à 139 ml d'une émulsion lipidique comprenant 17 g à 28 g de lipides, dans laquelle, si le patient souffre d'obésité de classe I ou II, 28 ml à 32 ml par kg d'IBW, de manière particulièrement préférée, 30 ml par kg d'IBW sont administrés au patient par jour, ou dans laquelle, si le patient souffre d'obésité de classe III, 35 ml à 39 ml par kg d'IBW, de manière particulièrement préférée, 37 ml par kg d'IBW sont administrés au patient par jour.

2. Solution de nutrition parentérale destinée à être utilisée selon la revendication 1, la solution de nutrition comprenant, pour 1000 ml :

   (i) 667 ml d'une solution d'acides aminés comprenant 67 g d'acides aminés,
   (ii) 222 ml d'une solution de glucose comprenant 89 g de glucose, et
   (iii) 111 ml d'une émulsion lipidique comprenant 22 g de lipides.

3. Solution de nutrition parentérale destinée à être utilisée selon l'une des revendications précédentes, dans laquelle la solution d'acides aminés comprend pour 1 000 ml de la solution d'acides aminés, au moins un constituant, choisi dans le groupe constitué de :

   environ 5 g d'isoleucine, environ 7,4 g de leucine, environ 9,31 g d'acétate de lysine (correspondant à environ 6,6 g de lysine), environ 4,3 g de méthionine, environ 5,1 g de phénylalanine, environ 4,4 g de thréonine, environ 2 g de tryptophane, environ 6,2 g de valine, environ 12 g d'arginine, environ 3 g d'histidine, environ 14 g d'alanine, environ 11 g de glycine, environ 11,2 g de proline, environ 6,5 g de sérine, environ 0,4 g de tyrosine 25 et environ 1 g de taurine ou une combinaison de ceux-ci,
   et/ou
   dans laquelle l'émulsion lipidique comprend, pour 1 000 ml d'émulsion lipidique, au moins un constituant, choisi dans le groupe constitué de :
   environ 60 g d'huile de soja, environ 60 g de MCT, environ 50 g d'huile d'olive et environ 30 g d'huile de poisson ou d'une combinaison de celles-ci.

4. Solution de nutrition parentérale destinée à être utilisée selon l'une des revendications précédentes, dans laquelle la solution de nutrition parentérale comprend en outre au moins un constituant, choisi dans le groupe constitué de : électrolytes, dipeptides, oligo-éléments, vitamines ou une combinaison de ceux-ci.

5. Solution de nutrition parentérale destinée à être utilisée selon la revendication 4, dans laquelle la solution de nutrition parentérale comprend, pour 1 000 ml de la solution de nutrition, des électrolytes choisis dans le groupe constitué d'environ 32,8 mmol de sodium à environ 48 mmol de sodium, d'environ 24 mmol de potassium à environ 36 mmol de potassium, d'environ 4,1 mmol de magnésium à environ 6,1 mmol de magnésium, d'environ 2 mmol de calcium à environ 3 mmol de calcium, d'environ 8,2 mmol de phosphate à environ 15,6 mmol de phosphate, d'environ 0,032 mmol de zinc à environ 0,048 mmol de zinc, d'environ 4,1 mmol de sulfate à environ 6,1 mmol de sulfate, d'environ 28,8 mmol de chlorure à environ 43,2 mmol de chlorure, d'environ 84,8 mmol d'acétate à environ 127,2 mmol d'acétate ou d'une combinaison de ceux-ci,
   dans laquelle de préférence la solution de nutrition parentérale comprend, pour 1 000 ml de la solution de nutrition, des électrolytes, choisis dans le groupe constitué d'environ 41 mmol de sodium, d'environ 30 mmol de potassium, d'environ 5,1 mmol de magnésium, d'environ 2,5 mmol de calcium, d'environ 13 mmol de phosphate, d'environ 0,04 mmol de zinc, d'environ 5,1 mmol de sulfate, d'environ 36 mmol de chlorure, d'environ 106 mmol d'acétate ou d'une combinaison de ceux-ci.

6. Solution de nutrition parentérale destinée à être utilisée selon l'une des revendications précédentes, dans laquelle la solution de nutrition comprend, pour 100 % d'énergie, 25 à 35 % d'énergie provenant des acides aminés, 40 à 45 % d'énergie provenant du glucose et 22 à 30 % d'énergie provenant des lipides, dans laquelle de préférence la solution de nutrition comprend, pour 100 % d'énergie, 28 à 34 % d'énergie provenant des acides aminés, 41 à 44 % d'énergie provenant du glucose et 25 à 27 % d'énergie provenant des lipides, dans laquelle le plus préférablement, la solution de nutrition comprend, pour 100 % d'énergie, 32 % d'énergie provenant des acides aminés, 42 % d'énergie provenant du glucose et 26 % d'énergie provenant des lipides.

7. Solution de nutrition parentérale destinée à être utilisée selon l'une des revendications précédentes, dans laquelle la solution de nutrition a une teneur énergétique de 700 kcal à 975 kcal pour 1000 ml, dans laquelle, de préférence, la solution de nutrition a une teneur énergétique de 800 kcal à 880 kcal pour 1000 ml, dans laquelle, le plus préférablement, la solution de nutrition a une teneur énergétique de 820 kcal à 860 kcal pour 1000 ml.

8. Solution de nutrition parentérale destinée à être utilisée selon l'une des revendications précédentes, dans laquelle la solution de nutrition parentérale est contenue dans une poche à trois chambres comprenant une première chambre, une deuxième chambre et une troisième chambre, avec

   (i) la première chambre comprenant la solution d'acides aminés,
   (ii) la deuxième chambre comprenant la solution de glucose, et
   (iii) la troisième chambre comprenant l'émulsion lipidique,

dans laquelle la solution d'acides aminés, la solution de glucose et l'émulsion lipidique sont combinées et mélangées avant utilisation.

9. Solution de nutrition parentérale destinée à être utilisée selon l'une des revendications précédentes, dans laquelle le patient en soins intensifs est un patient dans un état hypercatabolique et/ou hypermétabolique, dans laquelle de préférence le patient en soins intensifs est un patient, qui est gravement malade et/ou est un patient, qui a une pathologie, choisie dans le groupe constitué de : traumatisme, brûlure, infection, septicémie et opération chirurgicale ou une combinaison de ceux-ci.

10. Solution de nutrition parentérale destinée à être utilisée selon l'une des revendications précédentes, l'obésité est choisie dans le groupe constitué de : obésité de classe I, obésité de classe II et obésité de classe III.

11. Solution de nutrition parentérale destinée à être utilisée selon l'une des revendications précédentes, dans laquelle la quantité de la solution de nutrition parentérale à administrer quotidiennement est déterminée par un paramètre, choisi dans le groupe constitué de : taille du patient, sexe du patient, degré d'obésité ou une combinaison de ceux-ci.

12. Solution de nutrition parentérale destinée à être utilisée selon l'une des revendications précédentes,

   dans laquelle, si le patient est une femme et souffre d'obésité de classe I ou II, environ 2 100 ml de la solution de nutrition parentérale dans le cas d'une taille de 180 cm, environ 1 950 ml dans le cas d'une taille de 174 cm, environ 1 800 ml dans le cas d'une taille de 168 cm, environ 1 650 ml dans le cas d'une taille de 163 cm ou environ 1 500 ml dans le cas d'une taille de 157 cm doivent être administrés au patient par jour, ou
   dans laquelle, si le patient est une femme et souffre d'obésité de classe III, environ 2 590 ml de solution de nutrition parentérale dans le cas d'une taille de 180 cm, environ 2 405 ml dans le cas d'une taille de 174 cm, environ 2 220 ml dans le cas d'une taille de 168 cm, environ 2 035 ml dans le cas d'une taille de 163 cm ou environ 1 850 ml dans le cas d'une taille de 157 cm doivent être administrés au patient par jour, ou
   dans laquelle, si le patient est un homme et souffre d'obésité de classe I ou II, environ 2 550 ml de la solution de nutrition parentérale dans le cas d'une taille de 187 cm, environ 2 400 ml dans le cas d'une taille de 182 cm, environ 2 250 ml dans le cas d'une taille de 178 cm, environ 2 100 ml dans le cas d'une taille de 173 cm, environ 1 950 ml dans le cas d'une taille de 168 cm ou environ 1 800 ml dans le cas d'une taille de 163 cm doivent être administrés au patient par jour, ou
   dans laquelle, si le patient est un homme et souffre d'obésité de classe III, environ 3 145 ml de solution de nutrition parentérale dans le cas d'une taille de 187 cm, environ 2 960 ml dans le cas d'une taille de 182 cm, environ 2 775 ml dans le cas d'une taille de 178 cm, environ 2 590 ml dans le cas d'une taille de 173 cm, environ 2 405 ml dans le cas d'une taille de 168 cm ou environ 2 220 ml dans le cas d'une taille de 163 cm doivent être administrés au patient par jour.

13. Poche à trois chambres comprenant une première chambre, une deuxième chambre et une troisième chambre,

la première chambre comprenant 750 ml à 2 250 ml d'une solution d'acides aminés comprenant 56,3 g à 281,3 g d'acides aminés,
la deuxième chambre comprenant 250 ml à 750 ml d'une solution de glucose comprenant 75 g à 375 g de glucose, et
la troisième chambre comprenant 125 ml à 375 ml d'une émulsion lipidique comprenant 18,8 g à 93,8 g de lipides,
dans laquelle la poche à trois chambres, en fonction du volume total de la première, de la deuxième et de la troisième chambre, comprend au moins 50 g d'acides aminés pour 1 000 ml,
dans laquelle de préférence la poche à trois chambres, en fonction du volume total de la première, de la deuxième et de la troisième chambre, comprend au moins 60 g d'acides aminés pour 1 000 ml, de manière particulièrement préférée au moins 63 g d'acides aminés,
et dans laquelle l'émulsion lipidique, pour 1 000 ml, comprend au moins un composant, qui est choisi dans le groupe constitué d'environ 60 g d'huile de soja, d'environ 60 g de MCT, d'environ 50 g d'huile d'olive et d'environ 30 g d'huile de poisson ou d'une combinaison de ceux-ci.

14. Poche à trois chambres selon la revendication 13,

dans laquelle la première chambre comprend 1 500 ml d'une solution d'acides aminés comprenant 135 g à 187,5 g d'acides aminés, de préférence 150 g d'acides aminés,
la deuxième chambre comprend 500 ml d'une solution de glucose comprenant 150 g à 250 g de glucose, de préférence 200 g de glucose,
et la troisième chambre comprend 250 ml d'une émulsion lipidique comprenant 37,5 g à 62,5 g de lipides, de préférence 50 g de lipides,
ou
dans laquelle la première chambre comprend 750 ml d'une solution d'acides aminés comprenant 67,5 g à 93,8 g d'acides aminés, de préférence 75 g d'acides aminés,
la deuxième chambre comprend 250 ml d'une solution de glucose comprenant 75 g à 125 g de glucose, de préférence 100 g de glucose,
et la troisième chambre comprend 125 ml d'une émulsion lipidique comprenant 18,8 g à 31,3 g de lipides, de préférence 25 g de lipides,
ou
dans laquelle la première chambre comprend 2 250 ml d'une solution d'acides aminés comprenant 202,5 g à 281 g d'acides aminés, de préférence 225 g d'acides aminés,
la deuxième chambre comprend 750 ml d'une solution de glucose comprenant 225 g à 375 g de glucose, de préférence 300 g de glucose,
et la troisième chambre comprend 375 ml d'une émulsion lipidique comprenant 56,3 g à 93,8 g de lipides, de préférence 75 g de lipides.